# EUROPÄISCHE PATENTANMELDUNG

(11) **EP 1 029 854 A1**
(43) Veröffentlichungstag der Anmeldung: **23.08.2000**
(21) Anmeldenummer: 00101605.4
(22) Anmeldetag: 27.01.2000
(51) Int. Cl.: C07D 263/24, C07D 498/04, C07D 471/04, A61K 31/422, A61K 31/5383, A61P 31/04

(54) **Oxazolidinone und ihre Verwendung als antibakterielle Mittel**

(30) Priorität: 09.02.1999 DE 19905278
(71) Anmelder: BAYER AG, 51368 Leverkusen (DE)
(72) Erfinder: Bartel, Stephan, Dr., 51515 Kürten (DE); Raddatz, Siegfried, Dr., 51065 Köln (DE); Härter, Michael, Dr., 51375 Leverkusen (DE); Rosentreter, Ulrich, Dr., 42349 Wuppertal (DE); Wild, Hanno, Dr., 42113 Wuppertal (DE); Endermann, Rainer, Dr., 42113 Wuppertal (DE); Kroll, Hein-Peter, Dr., 42115 Wuppertal (DE)

(57) **Zusammenfassung**

Die vorliegende Erfindung betrifft neue substituierte Oxazolidinone der Formel (I), 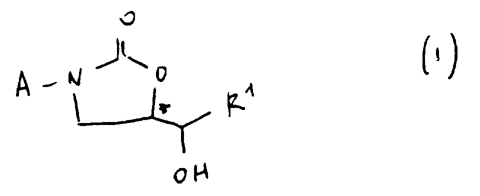 Verfahren zu ihrer Herstellung und ihre Verwendung als Arzneimittel, insbesondere als antibakterielle Arzneimittel.

## Beschreibung

Die vorliegende Erfindung betrifft neue substituierte Oxazolidinone, Verfahren zu ihrer Herstellung und ihre Verwendung als Arzneimittel, insbesondere als antibakterielle Arzneimittel.

Aus den Publikationen US 5 254 577, US 4 705 799, EP 311 090, EP 312 000 und C.H. Park et al., J. Med. Chem. 35, 1156 (1992), sind N-Aryloxazolidinone mit antibakterieller Wirkung bekannt. Außerdem sind 3-(Stickstoff-substituierte)phenyl-5-beta-amidomethyloxazolidin-2-one aus der EP 609 905 A1 bekannt.

Ferner sind in der EP 609 441 und EP 657 440 Oxazolidinonderivate mit einer Monoaminoxidase-inhibitorischen Wirkung und in der EP 645 376 mit Wirkung als Adhäsionsrezeptor-Antagonisten publiziert.

Desweiteren sind aus der EP-A-0738726 und aus WO-9737980-A Hydroxymethylsubstituierte Oxazolidinone bekannt.

Die Erfindung betrifft neue Hydroxymethyl-substituierte Oxazolidinone der allgemeinen Formel (I): 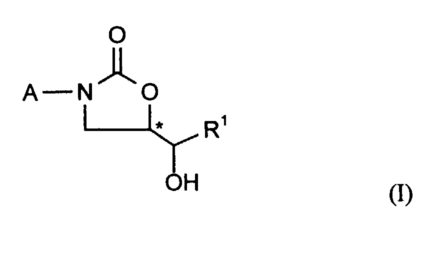 in welcher
R¹ geradkettiges oder verzweigtes Alkyl mit bis zu 6 Kohlenstoffatomen ist, das gegebenfalls mit 1 bis 3 Substituenten substituiert ist, die aus der Gruppe ausgewählt werden, die besteht aus Halogen, Cyano, Hydroxy, geradkettigem oder verzweigtem Alkoxy mit 2 bis 6 Kohlenstoffatomen, Mercapto, geradkettigem oder verzweigtem Alkylthio mit 2 bis 6 Kohlenstoffatomen, Aryl mit 6 bis 10 Kohlenstoffatomen und -NR²R³, worin
   R² und R³ Wasserstoff bedeuten, oder
   R² Wasserstoff bedeutet, und
   R³ einen Rest der Formeln 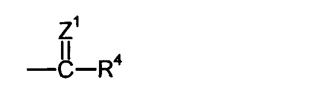 bedeutet,
      worin
      Z¹ ein Sauerstoff- oder Schwefelatom bedeutet,
      R⁴ geradkettiges oder verzweigtes Alkoxy mit bis zu 8 Kohlenstoffatomen oder Trifluormethyl bedeutet, oder
         Cycloalkyl mit 3 bis 6 Kohlenstoffatomen bedeutet, das gegebenenfalls durch Halogen oder Aryl mit 6 bis 10 Kohlenstoffatomen substituiert ist, oder
         Aryl mit 6 bis 10 Kohlenstoffatomen oder einen 5- bis 6-gliedrigen gesättigten oder aromatischen Heterocylcus mit bis zu 3 Heteroatomen aus der Reihe S, N und/oder O bedeutet, wobei die unter R⁴ aufgeführten Ringsysteme gegebenenfalls bis zu 2-fach gleich oder verschieden durch Halogen, Cyano, Nitro, Hydroxy oder Phenyl substituiert sind,
         oder
      R⁴ geradkettiges oder verzweigtes Alkyl mit bis zu 6 Kohlenstoffatomen bedeutet, das gegebenenfalls durch Phenoxy, Benzyloxy, Carboxyl, Halogen oder geradkettiges oder verzweigtes Alkoxycarbonyl oder Acyl mit jeweils bis zu 6 Kohlenstoffatomen oder durch einen 5-bis 6-gliedrigen Heterocyclus aus der Reihe S, N und/oder O substituiert ist,
         oder
      R⁴ einen Rest der Formel -NR⁵R⁶ bedeutet,
         worin
         R⁵ und R⁶ gleich oder verschieden sind und Wasserstoff, Phenyl, Pyridyl oder geradkettiges oder verzweigtes Alkyl mit bis zu 5 Kohlenstoffatomen bedeuten, das gegebenenfalls durch über N-gebundenes Morpholin substituiert ist, oder
R¹ geradkettiges oder verzweigtes Alkenyl mit 2 bis 6 Kohlenstoffatomen; geradkettiges oder verzweigtes Alkinyl mit 2 bis 6 Kohlenstoffatomen; geradkettiges oder verzweigtes Alkoxycarbonyl mit bis zu 6 Kohlenstoffatomen; Aryl mit 6 bis 10 Kohlenstoffatomen oder-CONR² 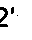 R³ 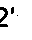 ist, worin R² 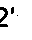 und R³ 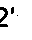 die oben angegebene Bedeutungen von R² und R³ aufweisen, oder
A für einen Rest der Formeln 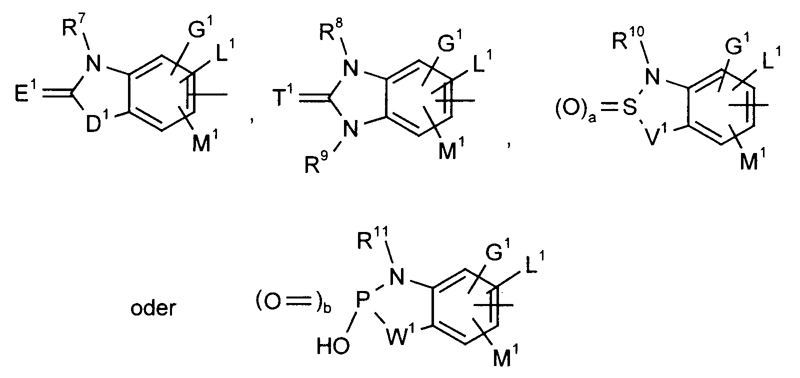 steht, worin
   G¹, L¹ und M¹ gleich oder verschieden sind und
      für Wasserstoff, Carboxy, Halogen, Cyano, Formyl, Trifluormethyl, Nitro, für geradkettiges oder verzweigtes Alkyl mit bis zu 6 Kohlenstoffatomen oder für eine Gruppe der Formel -CO-NR¹²R¹³ stehen,
      worin
      R¹² und R¹³ gleich oder verschieden sind und Wasserstoff, geradkettiges oder verzweigtes Alkyl mit bis zu 4 Kohlenstoffatomen oder Phenyl bedeuten,
   R⁷ Wasserstoff, Cycloalkylcarbonyl oder Cycloalkyl mit jeweils 3 bis 6 Kohlenstoffatomen, oder geradkettiges oder verzweigtes Alkoxycarbonyl mit bis zu 6 Kohlenstoffatomen, oder geradkettiges oder verzweigtes Alkyl oder Alkenyl mit jeweils bis zu 10 Kohlenstoffatomen bedeutet, das gegebenenfalls durch Cyano, Azido, Trifluormethyl, Pyridyl, Halogen, Hydroxy, Carboxyl, geradkettiges oder verzweigtes Alkoxycarbonyl mit bis zu 6 Kohlenstoffatomen, Aryl mit 6 bis 10 Kohlenstoffatomen, Cycloalkyl mit 3 bis 6 Kohlenstoffatomen und/oder durch eine Gruppe der Formel -(CO)_{c}-NR¹⁴R¹⁵, R¹⁶-N-SO₂-R¹⁷, R¹⁸R¹⁹-N-SO₂-, R²⁰-S(O)_{d} oder 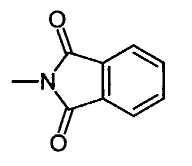 substituiert ist,
      worin
   c eine Zahl 0 oder 1 bedeutet,
   R¹⁴, R¹⁵, R¹⁶, R¹⁸ und R¹⁹, die oben angegebene Bedeutung von R¹² und R¹³ haben und mit dieser gleich oder verschieden sind,
      oder gemeinsam mit dem Stickstoffatom einen 5- bis 6-gliedrigen, gesättigten Heterocyclus mit gegebenenfalls einem weiteren Heteroatom aus der Serie N, S und/oder O bilden, der seinerseits gegebenenfalls, auch an einem weiteren Stickstoffatom, durch geradkettiges oder verzweigtes Alkyl oder Acyl mit bis zu 3 Kohlenstoffatomen substituiert sein kann,
   d eine Zahl 0, 1 oder 2 bedeutet,
   R¹⁷ und R²⁰ gleich oder verschieden sind und geradkettiges oder verzweigtes Alkyl mit bis zu 4 Kohlenstoffatomen, Benzyl, Phenyl oder Tolyl bedeuten,
   oder R⁷ einen Rest der Formeln 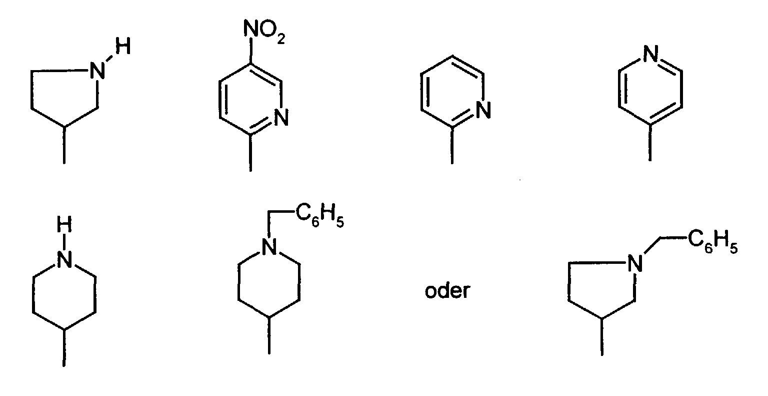 bedeutet oder
   R⁷ eine Gruppe der Formel -COCl₃ oder geradkettiges oder verzweigtes Acyl mit bis zu 6 Kohlenstoffatomen bedeutet, das gegebenenfalls durch -CF₃, -CCl₃ oder eine Gruppe der Formel -OR²¹ substituiert ist,
      worin
      R²¹ Wasserstoff oder geradkettiges oder verzweigtes Alkyl mit bis zu 6 Kohlenstoffatomen bedeutet, das gegebenenfalls durch Aryl mit bis zu 10 Kohlenstoffatomen substituiert ist,
         oder
      R⁷ eine Gruppe der Formel -(CO)ₑ-NR²²R²³, -NR²⁴-SO₂R²⁵, R²⁷R²⁶N-SO₂ oder R²⁸-S(O)_{f} bedeutet,
         worin
      e die oben angegebene Bedeutung von c hat und mit dieser gleich oder verschieden ist,
      R²², R²³ und R²⁴ die oben angegebene Bedeutung von R¹⁴, R¹⁵ und R¹⁶ haben und mit dieser gleich oder verschieden sind,
      R²⁶ und R²⁷ die oben angegebene Bedeutung von R¹² und R¹³ haben und mit dieser gleich oder verschieden sind,
      f die oben angegebene Bedeutung von d hat und mit dieser gleich oder verschieden ist,
      R²⁵ und R²⁸ die oben angegebene Bedeutungen von R¹⁷ und R¹⁹ haben und mit dieser gleich oder verschieden sind,
   D¹ ein Sauerstoff oder Schwefelatom bedeutet,
   E¹ ein Sauerstoff- oder Schwefelatom oder eine Gruppe der Formel NH bedeutet,
   T¹ ein Sauerstoffatom oder die NH-Gruppe bedeutet,
   R⁸ und R⁹ die oben angegebene Bedeutung von R⁷ haben und mit dieser gleich oder verschieden sind,
      oder
   T¹ ein Schwefelatom bedeutet,
   mit der Maßgabe, daß R⁸ und R⁹ die oben angegebene Bedeutung von R⁷ haben, aber nicht für Wasserstoff stehen,
   oder im Fall, daß R⁷, R⁸ und R⁹ nicht für Wasserstoff stehen, E¹ und/oder T¹ eine Gruppe der Formel NR²⁹ bedeuten, worin R²⁹ mit Ausnahme von Wasserstoff die oben angegebene Bedeutung von R⁷ hat und mit dieser gleich oder verschieden ist,
   oder R²⁹ Cyano oder eine Gruppe der Formel -CO₂R³⁰ bedeutet, worin R³⁰ Benzyl oder Phenyl bedeutet, die gegebenefalls durch Nitro oder Halogen substituiert sind
   V¹ und W¹ die oben angegebene Bedeutung von D¹ haben oder die oben aufgeführte Gruppe N-R⁹ bedeuten und mit dieser gleich oder verschieden sind,
   a eine Zahl 1 oder 2 bedeutet,
   b eine Zahl 0 oder 1 bedeutet,
   R¹⁰ und R¹¹ die oben angegebene Bedeutung von R⁷ haben und mit dieser gleich oder verschieden sind, oder
A für Reste der Formeln 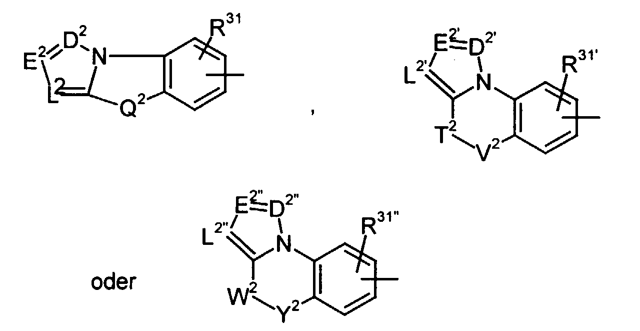 steht,
   worin
   R³¹, R^{31'} und R^{31''} gleich oder verschieden sind und Wasserstoff oder Halogen bedeuten,
   D², D²' und D²'' gleich oder verschieden sind und ein Stickstoffatom oder einen Rest der Formel CR³² bedeuten,
      worin
      R³² Wasserstoff, Trifluormethyl, Halogen, Hydroxy, geradkettiges oder verzweigtes Alkoxy mit bis zu 6 Kohlenstoffatomen oder einen Rest der Formel -NR³³R³⁴ bedeutet,
         worin
         R³³ und R³⁴ gleich oder verschieden sind und Wasserstoff, Aryl mit 6 bis 10 Kohlenstoffatomen, Benzyl oder geradkettiges oder verzweigtes Alkyl oder Acyl mit jeweils bis zu 6 Kohlenstoffatomen bedeuten,
   E², E²' und E²'' gleich oder verschieden sind und ein Stickstoffatom oder einen Rest der Formel CR³⁵ bedeuten,
      worin
      R³⁵ Wasserstoff, Trifluormethyl, Nitro, Cyano oder Halogen bedeutet, oder
         geradkettiges oder verzweigtes Alkyl mit bis zu 6 Kohlenstoffatomen oder Benzyl bedeutet, die gegebenenfalls durch Hydroxy oder durch geradkettiges oder verzweigtes Alkoxy mit bis zu 4 Kohlenstoffatomen substituiert sind, oder
         Aryl mit 6 bis 10 Kohlenstoffatomen oder einen aromatischen Heterocyclus mit bis zu 3 Heteroatomen aus der Reihe S, N und/oder O bedeutet, wobei die Ringsysteme gegebenenfalls bis zu 3-fach gleich oder verschieden durch Halogen, Hydroxy, Nitro, geradkettiges oder verzweigtes Alkyl mit bis zu 4 Kohlenstoffatomen oder Trifluormethyl substituiert sind, oder
      R³⁵ Reste der Formeln O-R³⁶, -CO-R³⁷ oder -NR³⁸R³⁹ bedeutet,
         worin
         R³⁶ Wasserstoff, Benzoyl, geradkettiges oder verzweigtes Alkyl oder Acyl mit jeweils bis zu 6 Kohlenstoffatomen, Benzyl oder Aryl mit 6 bis 10 Kohlenstoffatomen oder einen aromatischen Heterocyclus mit bis zu 3 Heteroatomen aus der Reihe S, N und/oder O bedeutet,
         R³⁷ Hydroxy, geradkettiges oder verzweigtes Alkyl oder Alkoxy mit jeweils bis zu 6 Kohlenstoffatomen, Benzyl oder Aryl mit 6 bis 10 Kohlenstoffatomen oder einen aromatischen Heterocyclus mit bis zu 3 Heteroatomen aus der Reihe S, N und/oder O bedeutet,
            oder
         R³⁷ eine Gruppe der Formel -NR⁴⁰R⁴¹ bedeutet,
            worin
            R⁴⁰ und R⁴¹ gleich oder verschieden sind und Wasserstoff, Aryl mit 6 bis 10 Kohlenstoffatomen, Benzyl oder geradkettiges oder verzweigtes Alkyl mit bis zu 6 Kohlenstoffatomen bedeuten,
         R³⁸ und R³⁹ gleich oder verschieden sind und Wasserstoff, Benzyl, Aryl mit 6 bis 10 Kohlenstoffatomen, geradkettiges oder verzweigtes Alkyl mit bis zu 6 Kohlenstoffatomen oder eine Gruppe der Formel -CO₂R⁴² oder -CM²-NR⁴³R⁴⁴ bedeuten,
            worin
            R⁴² geradkettiges oder verzweigtes Alkyl mit bis zu 6 Kohlenstoffatomen, Benzyl oder Phenyl bedeutet,
            M² ein Sauerstoff- oder Schwefelatom bedeutet,
            R⁴³ und R⁴⁴ gleich oder verschieden sind und die oben angegebene Bedeutung von R³³ und R³⁴ haben,
            oder
         R³⁸ Wasserstoff bedeutet
            und
         R³⁹ einen Rest der Formel 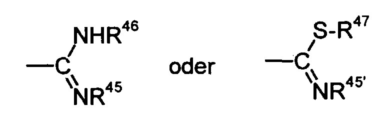 bedeutet,
            worin
            R⁴⁵ und R^{45'} gleich oder verschieden sind und Wasserstoff, geradkettiges oder verzweigtes Alkyl mit bis zu 6 Kohlenstoffatomen, Aryl mit 6 bis 10 Kohlenstoffatomen oder Benzyl bedeuten,
            R⁴⁶ und R⁴⁷ gleich oder verschieden sind und geradkettiges oder verzweigtes Alkyl mit bis zu 6 Kohlenstoffatomen, Phenyl oder Benzyl bedeuten,
   L², L²' und L²'' gleich oder verschieden sind und ein Stickstoffatom oder einen Rest der Formel CR⁴⁸ bedeuten,
      worin
      R⁴⁸ Wasserstoff, Trifluormethyl, Nitro, Cyano, Halogen oder geradkettiges oder verzweigtes Alkyl mit bis zu 6 Kohlenstoffatomen bedeutet, das gegebenenfalls durch Phenyl oder durch einen Rest der Formel -OR⁴⁹ substituiert ist,
         worin
         R⁴⁹ Wasserstoff, geradkettiges oder verzweigtes Alkyl mit bis zu 6 Kohlenstoffatomen, Aryl mit 6 bis 10 Kohlenstoffatomen oder Benzyl bedeutet,
         oder
      R⁴⁸ Reste der Formeln -OR⁵⁰, -COR⁵¹ oder -NR⁵²R⁵³ bedeutet,
         worin
         R⁵⁰ Wasserstoff, Aryl mit 6 bis 10 Kohlenstoffatomen, Benzyl oder geradkettiges oder verzweigtes Alkyl oder Acyl mit jeweils bis zu 6 Kohlenstoffatomen bedeutet,
         R⁵¹ die oben angegebene Bedeutung von R³⁹ hat und mit dieser gleich oder verschieden ist,
         R⁵² und R⁵³ die oben angegebene Bedeutung von R³³ und R³⁴ haben und mit diesen gleich oder verschieden sind,
            oder
         R⁵² Wasserstoff bedeutet
            und
         R⁵³ Cyano oder einen Rest der Formel -CO-NR⁵⁴R⁵⁵ oder -CS-NR⁵⁶R⁵⁷ bedeutet,
            worin
            R⁵⁴, R⁵⁵, R⁵⁶ und R⁵⁷ gleich oder verschieden sind und die oben angegebene Bedeutung von R³⁵ und R³⁶ haben,
            oder
         R⁵² und R⁵³ gemeinsam mit dem Stickstoffatom einen 5- bis 6-gliedrigen, gesättigten Heterocyclus bilden, der noch ein weiteres Heteroatom aus der Reihe S, O oder einen Rest der Formel -NH enthalten kann,
   Q² ein Sauerstoff- oder Schwefelatom oder Reste der Formeln SO₂, SO, C=O oder CR⁵⁸R⁵⁹ bedeutet,
      worin
      R⁵⁸ und R⁵⁹ gleich oder verschieden sind und Wasserstoff oder Halogen bedeuten,
   T² einen Rest der Formel CR⁶⁰R⁶¹ bedeutet,
      worin
      R⁶⁰ und R⁶¹ gleich oder verschieden sind und Wasserstoff, Halogen, Hydroxy, geradkettiges oder verzweigtes Alkyl oder Alkoxy mit jeweils bis zu 6 Kohlenstoffatomen oder Benzyloxy bedeuten,
         oder
      R⁶⁰ und R⁶¹ gemeinsam Reste der Formeln =O, =S, 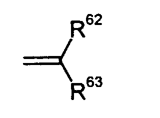 oder =N-R⁶⁴ bilden,
         worin
         R⁶² und R⁶³ gleich oder verschieden sind und Wasserstoff, geradkettiges oder verzweigtes Alkyl mit bis zu 6 Kohlenstoffatomen oder Benzyl bedeuten,
            oder
         R⁶² und R⁶³ gemeinsam einen 3- bis 6-gliedrigen, gesättigten oder partiell ungesättigten Carbocyclus bilden,
            und
         R⁶⁴ Wasserstoff, Benzyl oder geradkettiges oder verzweigtes Alkyl oder Acyl mit jeweils bis zu 6 Kohlenstoffatomen bedeutet,
   V² ein Sauerstoffatom, ein Schwefelatom oder einen Rest der Formel SO oder SO₂ bedeutet,
   W² ein Sauerstoff- oder Schwefelatom bedeutet, oder Reste der Formeln C=O, C=S, SO, SO₂, NR⁶⁵ oder CR⁶⁶R⁶⁷ bedeutet,
      worin
      R⁶⁵ die oben angegebene Bedeutung von R⁶⁴ hat und mit dieser gleich oder verschieden ist,
      R⁶⁶ und R⁶⁷ gleich oder verschieden sind und Wasserstoff, Halogen, geradkettiges oder verzweigtes Alkyl mit bis zu 6 Kohlenstoffatomen oder Benzyl bedeuten,
         oder
      R⁶⁶ Wasserstoff bedeutet
         und
      R⁶⁷ einen Rest der Formel -OR⁶⁸ bedeutet,
         worin
         R⁶⁸ Wasserstoff, geradkettiges oder verzweigtes Alkyl oder Acyl mit jeweils bis zu 6 Kohlenstoffatomen oder Benzyl bedeutet,
   Y² einen Rest der Formel C=O oder -CR⁶⁹R⁷⁰ bedeutet,
      worin
      R⁶⁹ und R⁷⁰ gleich oder verschieden sind und Wasserstoff, Halogen, Benzyl oder geradkettiges oder verzweigtes Alkyl mit bis zu 6 Kohlenstoffatomen bedeuten,
         oder
      R⁶⁹ Wasserstoff bedeutet
         und
      R⁷⁰ Hydroxy, Benzyloxy oder geradkettiges oder verzweigtes Alkoxy mit bis zu 6 Kohlenstoffatomen bedeutet,
      oder
   W² und Y gemeinsam für die Gruppe -CH=CH- stehen, oder
A für einen Rest der Formel 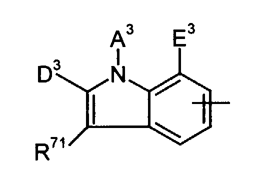 steht, in welcher
R⁷¹ für Wasserstoff, Halogen oder für geradkettiges oder verzweigtes Alkyl mit bis zu 4 Kohlenstoffatomen steht,
E³ für Wasserstoff oder für Halogen steht,
A³ und D³ gemeinsam unter Einbezug des Stickstoffatoms einen heterocyclischen Rest der Formel 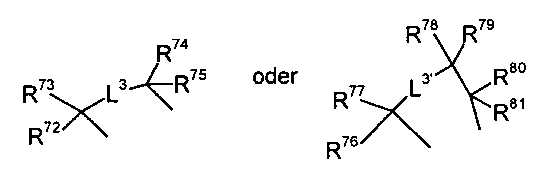 bilden,
   worin
L³ und L³' gleich oder verschieden sind und ein Sauerstoffatom oder einen Rest der Formel -NR⁸² bedeuten,
   worin
R⁸² Wasserstoff, Carboxyl, Cycloalkyl mit 3 bis 6 Kohlenstoffatomen, geradkettiges oder verzweigtes Alkoxycarbonyl mit bis zu 6 Kohlenstoffatomen bedeutet, oder
   geradkettiges oder verzweigtes Acyl mit bis zu 6 Kohlenstoffatomen bedeutet, das gegebenenfalls durch Halogen, Hydroxy, geradkettiges oder verzweigtes Alkoxy mit bis zu 5 Kohlenstoffatomen oder durch eine Gruppe der Formel -NR⁸³R⁸⁴ substituiert ist,
   worin
   R⁸³ und R⁸⁴ gleich oder verschieden sind und Wasserstoff, Phenyl oder geradkettiges oder verzweigtes Alkyl mit bis zu 5 Kohlenstoffatomen bedeuten,
      oder
      geradkettiges oder verzweigtes Alkyl oder Alkenyl mit jeweils bis zu 6 Kohlenstoffatomen bedeutet, die gegebenenfalls durch Cyano, Halogen, Carboxyl, geradkettiges oder verzweigtes Alkoxycarbonyl mit bis zu 5 Kohlenstoffatomen oder durch eine Gruppe der Formel -N^{83'}R^{84'} substituert sind,
      worin
   R^{83'} und R^{84'} die oben angegebene Bedeutung von R⁸³ und R⁸⁴ haben und mit dieser gleich oder verschieden sind,
      und/oder Alkyl oder Alkenyl gegebenenfalls durch Aryl mit 6 bis 14 Kohlenstoffatomen substituiert sind, das seinerseits durch Halogen oder durch geradkettiges oder verzweigtes Alkyl, Alkoxy oder Acyl mit jeweils bis zu 6 Kohlenstoffatomen substituiert sein kann,
R⁷², R⁷³, R⁷⁴ und R⁷⁵ gleich oder verschieden sind und Wasserstoff oder geradkettiges oder verzweigtes Alkyl mit bis zu 6 Kohlenstoffatomen bedeuten, das gegebenenfalls durch Halogen substituiert ist,
   oder
R⁷² und R⁷³ und/oder R⁷⁴ und R⁷⁵ gemeinsam Reste der Formel =O, oder =S bilden,
R⁷⁶, R⁷⁷, R⁷⁸, R⁷⁹, R⁸⁰ und R⁸¹ gleich oder verschieden sind und Wasserstoff oder geradkettiges oder verzweigtes Alkyl mit bis zu 6 Kohlenstoffatomen bedeuten, das gegebenenfalls durch Hydroxy, Halogen, geradkettiges oder verzweigtes Alkoxy oder Alkoxycarbonyl mit jeweils bis zu 6 Kohlenstoffatomen, Aryl mit 6 bis 10 Kohlenstoffatomen oder durch eine Gruppe der Formel -NR⁸⁵R⁸⁶ substituiert ist,
   worin
   R⁸⁵ und R⁸⁶ die oben angegebene Bedeutung von R⁸³ und R⁸⁴ haben und mit dieser gleich oder verschieden sind,
   oder
R⁷⁶ und R⁷⁷ und/oder R⁷⁸ und R⁷⁹ und/oder R⁸⁰ und R⁸¹ gemeinsam Reste der Formel =O oder =S bilden
   und/oder
R⁷⁹ und R⁸⁰ gemeinsam eine endocyclische Doppelbindung bilden, oder
A für Reste der Formeln 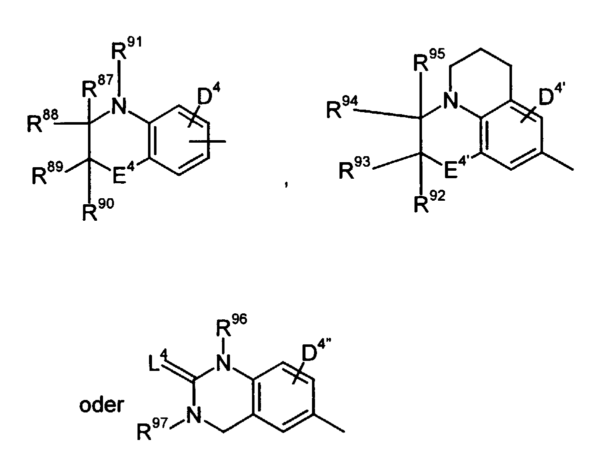 steht,
   steht worin
   D⁴, D⁴' und D⁴'' gleich oder verschieden sind und Wasserstoff, Carboxy, Halogen, Cyano, Formyl, Trifluormethyl, Nitro, geradkettiges oder verzweigtes Alkoxy, Alkoxycarbonyl, Alkylthio oder Acyl mit jeweils bis zu 6 Kohlenstoffatomen oder geradkettiges oder verzweigtes Alkyl mit bis zu 6 Kohlenstoffatomen bedeuten,
   E⁴ und E⁴' gleich oder verschieden sind und eine -CH₂-Gruppe, ein Sauerstoff- oder Schwefelatom oder einen Rest der Formel -SO oder -SO₂ bedeuten,
   L⁴ ein Sauerstoff- oder Schwefelatom oder eine Gruppe der Formel =NR⁹⁸ bedeutet, worin
      R⁹⁸ Wasserstoff, Phenyl, Hydroxy oder geradkettiges oder verzweigtes Alkoxy mit bis zu 6 Kohlenstoffatomen bedeutet,
   R⁸⁷, R⁸⁸, R⁸⁹, R⁹⁰, R⁹², R⁹³, R⁹⁴ und R⁹⁵ gleich oder verschieden sind und Wasserstoff oder geradkettiges oder verzweigtes Alkyl oder Alkenyl mit jeweils bis zu 6 Kohlenstoffatomen bedeuten, die gegebenenfalls durch Aryl mit 6 bis 10 Kohlenstoffatomen oder durch einen 5- bis 6-gliedrigen aromatischen Heterocyclus mit bis zu 3 Heteroatomen aus der Reihe S, N und/oder O substituiert sind, die ihrerseits ein- bis mehrfach durch geradkettiges oder verzweigtes Alkoxy oder Alkyl mit jeweils bis zu 6 Kohlenstoffatomen, Hydroxy oder Halogen substituiert sein können,
      oder
   R⁸⁷ und R⁸⁸, R⁸⁹ und R⁹⁰, R⁹² und R⁹³ und/oder R⁹⁴ und R⁹⁵ gemeinsam Gruppen der Formel =O, =CH₂ oder =CHR⁹⁹ bilden,
      worin
      R⁹⁹ Aryl mit 6 bis 10 Kohlenstoffatomen oder einen 5- bis 7-gliedrigen aromatischen Heterocyclus mit bis zu 3 Heteroatomen aus der Reihe S, N und/oder O bedeutet, wobei die Ringsysteme gegebenenfalls ein- bis mehrfach durch Halogen, Hydroxy oder durch geradkettiges oder verzweigtes Alkyl oder Alkoxy mit jeweils bis zu 6 Kohlenstoffatomen substituiert sind,
   R⁹¹, R⁹⁶ und R⁹⁷ gleich oder verschieden sind und Wasserstoff, geradkettiges oder verzweigtes Alkyl oder Alkoxycarbonyl mit jeweils bis zu 6 Kohlenstoffatomen bedeuten, oder
      einen Rest der Formel -CO-R¹⁰⁰ bedeuten,
      worin
      R¹⁰⁰ Aryl mit 6 bis 10 Kohlenstoffatomen, ein 5- bis 7-gliedriger aromatischer Heterocyclus mit bis zu 3 Heteroatomen aus der Reihe S, N und/oder O oder geradkettiges oder verzweigtes Alkyl mit bis zu 4 Kohlenstoffatomen bedeutet, das gegebenenfalls durch Aryl mit 6 bis 10 Kohlenstoffatomen substituiert ist, wobei die unter R¹⁰⁰ aufgeführten Ringsysteme gegebenfalls ein- bis mehrfach, gleich oder verschieden durch Halogen, Trifluormethyl, Nitro, Hydroxy oder durch geradkettiges oder verzweigtes Alkyl oder Alkoxy mit jeweils bis zu 6 Kohlenstoffatomen substituiert sind, oder
A eine Gruppe der Formel 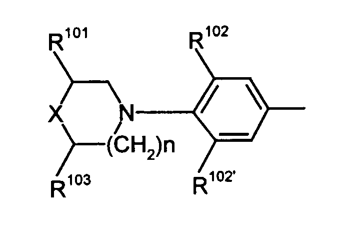 worin
   X O, S, SO, SO₂, S(O)NR¹⁰⁴ oder S(O)₂NR¹⁰⁴ ist;
   worin R¹⁰⁴ Wasserstoff, wahlweise durch ein bis drei Substituenten ausgewählt aus Chlor, Fluor, Hydroxy, (C₁-C₆)Alkoxy, Amino und Mono- oder Di(C₁-C₆)-Alkylamino substituiertes (C₁-C₄)Alkyl oder p-Toluolsulfonyl ist,
   R¹⁰¹ Wasserstoff ist, und,
      wenn X O ist, R¹⁰¹ Wasserstoff -CH₃, -CN, -CO₂H, -CO₂R¹⁰⁵, worin
      R¹⁰⁵ Wasserstoff, wahlweise durch ein bis drei Substituenten ausgewählt aus Chlor, Fluor, Hydroxy, (C₁-C₆)Alkoxy, (C₁-C₆)Acyloxy und -OCH₂-Ph substituiertes (C₁-C₄)Alkyl, (C₃-C₆)Cycloalkyl, Amino, Mono- oder Di(C₁-C₆)Alkylamino oder (C₁-C₆)Alkoxy ist, oder -(CH₂)_{g}R¹⁰⁶ ist, worin g 1 oder 2 und R¹⁰⁶ Wasserstoff, -OR¹⁰⁵, -OCOR¹⁰⁵, -NH₂, -NHCOR¹⁰⁵ oder -N(R¹⁰⁴)₂ ist, worin R¹⁰⁵ und R¹⁰⁵, die oben angegebenen Bedeutungen aufweisen, und
   R¹⁰² und R¹⁰² 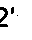 unabhängig voneinander Wasserstoff, Chlor oder Fluor bedeuten,
   R¹⁰³ Wasserstoff oder Methyl ist,
   n 0, 1 oder 2 ist
und tautomere Formen und/oder Salze davon.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweilige Mischungen. Die Racemformen lassen sich ebenso wie die Diastereomeren in bekannter Weise in die stereoisomer einheitlichen Bestandteile trennen.

Folgendes Formelschema veranschaulicht die entsprechend gekennzeichneten Schreibweisen für enantiomerenreine und racemische Formen:

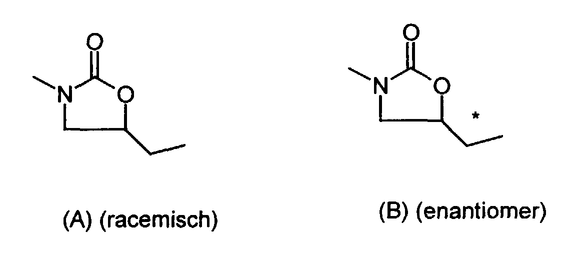

Desweiteren können die Verbindungen gegebenenfalls auch in tautomeren Formen vorliegen.

Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können weiterhin Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminem wie beispielsweise Diethylamin, Triethylamin, Ethyldiisopropylamin, Prokain, Dibenzylamin, N-Methylmorpholin, Dihydroabietylamin, 1-Ephenamin oder Methyl-piperidin.

Cycloalkyl steht im allgemeinen für einen cyclischen Kohlenwasserstoffrest mit 3 bis 8 Kohlenstoffatomen. Beispielsweise seien Cyclopropyl, Cyclobutyl, Cyclopentyl, Cyclohexyl, Cycloheptyl und Cyclooctyl genannt. Bevorzugt sind der Cyclopropyl-, Cyclopentan- und der Cyclohexanring.

Aryl steht im allgemeinen für einen aromatischen Rest mit 6 bis 10 Kohlenstoffatomen. Bevorzugte Arylreste sind Phenyl und Naphthyl.

Acyl bzw. (C₁-C₆)Acyl steht im Rahmen der Erfindung für einen geradkettigen oder verzweigten Acylrest mit 1 bis 6 Kohlenstoffatomen. Bevorzugt ist ein geradkettiger oder verzweigter Niedrigacylrest mit 1 bis 4 Kohlenstoffatomen. Bevorzugte Acylreste sind Acetyl und Propionyl.

Alkoxy bzw. (C₁-C₆)Alkoxy steht im Rahmen der Erfindung für einen geradkettigen oder verzweigten Alkoxyrest mit 1 bis 6 Kohlenstoffatomen. Bevorzugt ist ein geradkettiger oder verzweigter Niedrigalkoxyrest mit 1 bis 4 Kohlenstoffatomen. Beispielsweise seien genannt: Methoxy, Ethoxy, Propoxy, Isopropoxy, tert.-Butoxy, n-Pentoxy und n-Hexoxy.

Alkoxycarbonyl bzw. (C₁-C₆)Alkoxycarbonyl steht im Rahmen der Erfindung für einen geradkettigen oder verzweigten Alkoxycarbonylrest mit 1 bis 6 Kohlenstoffatomen. Bevorzugt ist ein geradkettiger oder verzweigter Niedrigalkoxycarbonylrest mit 1 bis 4 Kohlenstoffatomen. Beispielsweise seien genannt: Methoxycarbonyl, Ethoxycarbonyl, Propoxycarbonyl, Isopropoxycarbonyl, tert.-Butoxycarbonyl, n-Pentoxycarbonyl und n-Hexoxycarbonyl.

Bevorzugt sind in einer Ausführungsform der Erfindung Verbindungen, worin A für einen Rest der Formeln 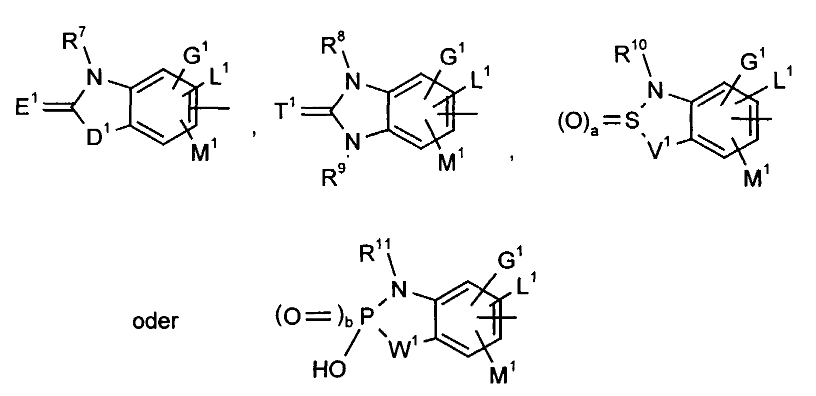 steht,
worin G¹, L¹ und M¹ für Wasserstoff stehen,
R⁷ Wasserstoff, Cyclopropylcarbonyl, Cyclopropyl, Cyclobutyl, Cyclopentyl, Cyclohexyl oder geradkettiges oder verzweigtes Alkoxycarbonyl mit bis zu 3 Kohlenstoffatomen bedeutet oder geradkettiges oder verzweigtes Alkyl oder Alkenyl mit bis zu 8 Kohlenstoffatomen bedeutet, die gegebenenfalls durch Cyano, Azido, Trifluormethyl, Pyridyl, Fluor, Chlor, Brom, Pyridyl, Hydroxy, Carboxyl, geradkettiges oder verzweigtes Alkoxycarbonyl mit bis zu 4 Kohlenstoffatomen, Phenyl, Benzyloxycarbonyl, Cyclopropyl, Cyclopentyl, Cyclohexyl, und/oder durch eine Gruppe der Formel -(CO)_{c}-NR¹⁴R¹⁵, R¹⁶-N-SO₂-R¹⁷, R¹⁸R¹⁹-N-SO₂-, R²⁰-S(O)_{d} oder 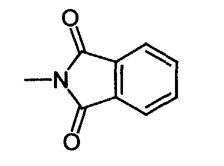 substituiert ist,
   c eine Zahl 0 oder 1 bedeutet,
      worin R¹⁴, R¹⁵, R¹⁶, R¹⁸ und R¹⁹ gleich oder verschieden sind und Wasserstoff, Methyl oder Ethyl bedeuten,
   d eine Zahl 0, 1 oder 2 bedeutet,
   R¹⁷ und R²⁰ gleich oder verschieden sind und geradkettiges oder verzweigtes Alkyl mit bis zu 4 Kohlenstoffatomen, Benzyl, Phenyl oder Tolyl bedeuten,
oder R⁷ einen Rest der Formeln 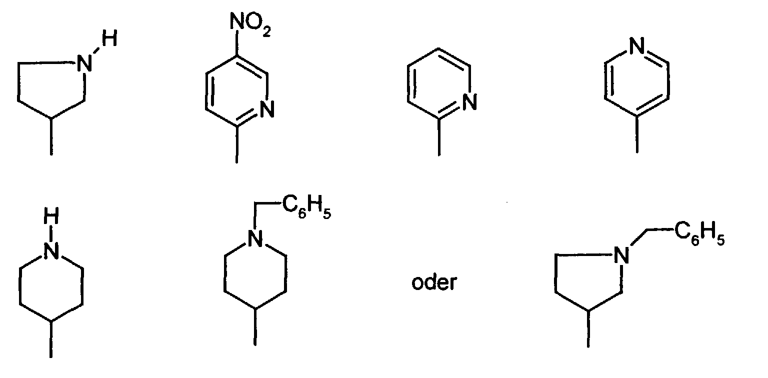 bedeutet oder
R⁷ eine Gruppe der Formel -COCl₃ oder geradkettiges oder verzweigtes Acyl mit bis zu 4 Kohlenstoffatomen bedeutet, das gegebenenfalls durch -CF₃, -CCl₃ oder eine Gruppe der Formel -OR²¹ substituiert ist, worin
   R²¹ Wasserstoff oder geradkettiges oder verzweigtes Alkyl mit bis zu 4 Kohlenstoffatomen bedeutet, das gegebenenfalls durch Phenyl substituiert ist,
      oder
   R⁷ eine Gruppe der Formel -(CO)_{c}-NR²²R²³ oder R²⁸-S(O)_{f} bedeutet,
      worin
   e die Zahl 1 ist,
   R²² und R²³ gleich oder verschieden sind und Wasserstoff, Methyl oder Ethyl bedeuten,
   f die oben angegebene Bedeutung von d hat und mit dieser gleich oder verschieden ist,
   R²⁸ Methyl, Phenyl, Tolyl oder Benzyl bedeutet,
D¹ ein Sauerstoff oder Schwefelatom bedeutet,
E¹ ein Sauerstoff- oder Schwefelatom oder eine Gruppe der Formel NH bedeutet,
T¹ ein Sauerstoffatom oder die NH-Gruppe bedeutet,
R⁸ und R⁹ die oben angegebene Bedeutung von R⁷ haben und mit dieser gleich oder verschieden sind,
   oder
T¹ ein Schwefelatom bedeutet,
mit der Maßgabe, daß R⁸ und R⁹ die oben angegebene Bedeutung von R⁷ haben, aber nicht für Wasserstoff stehen,
oder im Fall, daß R⁷, R⁸ und R⁹ nicht für Wasserstoff stehen, E¹ und/oder T¹ eine Gruppe der Formel NR²⁹ bedeuten, worin R²⁹ mit Ausnahme von Wasserstoff die oben angegebene Bedeutung von R⁷ hat und mit dieser gleich oder verschieden ist,
oder R²⁹ Cyano oder eine Gruppe der Formel -CO₂R³⁰ bedeutet, worin R³⁰ Benzyl oder Phenyl bedeutet, die gegebenenfalls durch Nitro oder Halogen substituiert sind;
V¹ und W¹ die oben angegebene Bedeutung von D¹ haben oder die oben aufgeführte Gruppe N-R⁹ bedeuten und mit dieser gleich oder verschieden sind,
a eine Zahl 1 oder 2 bedeutet,
b eine Zahl 0 oder 1 bedeutet,
R¹⁰ und R¹¹ die oben angegebene Bedeutung von R⁷ haben und mit dieser gleich oder verschieden sind,
   und tautomere Formen und/oder Salze davon.

Bevorzugt sind in einer weiteren Ausführungsform der Erfindung Verbindungen, worin A für einen Rest der Formeln, worin A ausgewählt wird aus der Gruppe der Formeln: 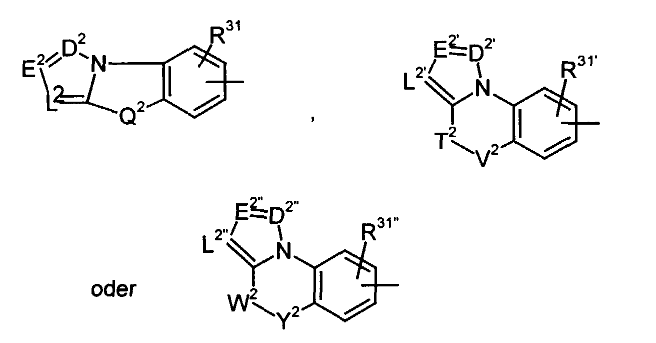 steht,
worin
R³¹, R^{31'} und R^{31''} gleich oder verschieden sind und Wasserstoff oder Fluor bedeuten,
D², D²' und D²'' gleich oder verschieden sind und ein Stickstoffatom oder einen Rest der Formel CR³² bedeuten,
   worin
   R³² Wasserstoff, Trifluormethyl, Chlor, Fluor, Hydroxy, geradkettiges oder verzweigtes Alkoxy mit bis zu 3 Kohlenstoffatomen bedeutet,
E², E²' und E²'' gleich oder verschieden sind und ein Stickstoffatom oder einen Rest der Formel CR³⁵ bedeuten,
   worin
   R³⁵ Wasserstoff, Trifluormethyl, Nitro, Cyano, Fluor, Chlor, geradkettiges oder verzweigtes Alkyl mit bis zu 3 Kohlenstoffatomen oder Benzyl bedeutet, die gegebenenfalls durch Hydroxy substituiert sind, oder Phenyl, Naphtyl, Pyridyl, Pyrimidyl, Pyrazinyl, Thienyl oder Furyl bedeutet, oder
   R³⁵ Reste der Formeln O-R³⁶, -CO-R³⁷ oder -NR³⁸R³⁹ bedeutet,
      worin
      R³⁶ Wasserstoff, geradkettiges oder verzweigtes Alkyl oder Acyl mit jeweils bis zu 4 Kohlenstoffatomen, Benzyl oder Phenyl bedeutet,
      R³⁷ Hydroxy, geradkettiges oder verzweigtes Alkyl oder Alkoxy mit jeweils bis zu 3 Kohlenstoffatomen, Benzyl oder Phenyl bedeutet,
         oder
      R³⁷ eine Gruppe der Formel -NR⁴⁰R⁴¹ bedeutet,
         worin
         R⁴⁰ und R⁴¹ gleich oder verschieden sind und Wasserstoff, Phenyl, Benzyl oder geradkettiges oder verzweigtes Alkyl mit bis zu 3 Kohlenstoffatomen bedeuten,
      R³⁸ und R³⁹ gleich oder verschieden sind und Wasserstoff, Benzyl, Phenyl, geradkettiges oder verzweigtes Alkyl mit bis zu 3 Kohlenstoffatomen oder eine Gruppe der Formel -CO₂R⁴² bedeuten,
         worin
         R⁴² geradkettiges oder verzweigtes Alkyl mit bis zu 3 Kohlenstoffatomen, Benzyl oder Phenyl bedeutet,
L², L²' und L²'' gleich oder verschieden sind und ein Stickstoffatom oder einen Rest der Formel CR⁴⁸ bedeuten,
   worin
   R⁴⁸ Wasserstoff, Trifluormethyl, Nitro, Cyano, Fluor, Chlor oder geradkettiges oder verzweigtes Alkyl mit bis zu 3 Kohlenstoffatomen bedeutet, das gegebenenfalls durch Phenyl oder durch einen Rest der Formel -OR⁴⁹ substituiert ist,
      worin
      R⁴⁹ Wasserstoff, geradkettiges oder verzweigtes Alkyl mit bis zu 6 Kohlenstoffatomen, Aryl mit 6 bis 10 Kohlenstoffatomen oder Benzyl bedeutet,
      oder
   R⁴⁸ Reste der Formeln -OR⁵⁰, -COR⁵¹ oder -NR⁵²R⁵³ bedeutet,
      worin
      R⁵⁰ Wasserstoff, Phenyl, Benzyl oder geradkettiges oder verzweigtes Alkyl oder Acyl mit jeweils bis zu 3 Kohlenstoffatomen bedeutet,
      R⁵¹ die oben angegebene Bedeutung von R³⁹ hat und mit dieser gleich oder verschieden ist,
      R⁵² und R⁵³ die oben angegebene Bedeutung von R³³ und R³⁴ haben und mit diesen gleich oder verschieden sind,
Q² ein Sauerstoff- oder Schwefelatom oder Reste der Formeln SO₂, SO, C=O oder CR⁵⁸R⁵⁹ bedeutet,
   worin
   R⁵⁸ und R⁵⁹ gleich oder verschieden sind und Wasserstoff oder Fluor bedeuten,
T² einen Rest der Formel -CR⁶⁰R⁶¹ bedeutet,
   worin
   R⁶⁰ und R⁶¹ gleich oder verschieden sind und Wasserstoff, Fluor, Hydroxy, geradkettiges oder verzweigtes Alkyl oder Alkoxy mit jeweils bis zu 3 Kohlenstoffatomen oder Benzyloxy bedeuten,
      oder
   R⁶⁰ und R⁶¹ gemeinsam Reste der Formeln =O oder =S bilden,
V² ein Sauerstoffatom, ein Schwefelatom oder einen Rest der Formel SO oder SO₂ bedeutet,
W² ein Sauerstoff- oder Schwefelatom bedeutet, oder Reste der Formeln C=O, C=S, SO, SO₂, NR⁶⁵ oder CR⁶⁶R⁶⁷ bedeutet,
   worin
   R⁶⁵ Wasserstoff, Benzyl oder geradkettiges oder verzweigtes Alkyl oder Acyl mit jeweils bis zu 3 Kohlenstoffatomen bedeutet,
   R⁶⁶ und R⁶⁷ gleich oder verschieden sind und Wasserstoff, Fluor, geradkettiges oder verzweigtes Alkyl mit bis zu 3 Kohlenstoffatomen oder Benzyl bedeuten, oder
Y² einen Rest der Formel C=O oder -CR⁶⁹R⁷⁰ bedeutet,
   worin
   R⁶⁹ und R⁷⁰ gleich oder verschieden sind und Wasserstoff, Fluor, Benzyl oder geradkettiges oder verzweigtes Alkyl mit bis zu 6 Kohlenstoffatomen bedeuten,
   und tautomere Formen und/oder Salze davon.

Bevorzugt sind in einer weiteren Ausführungsform der Erfindung Verbindungen, worin A für einen Rest der Formeln 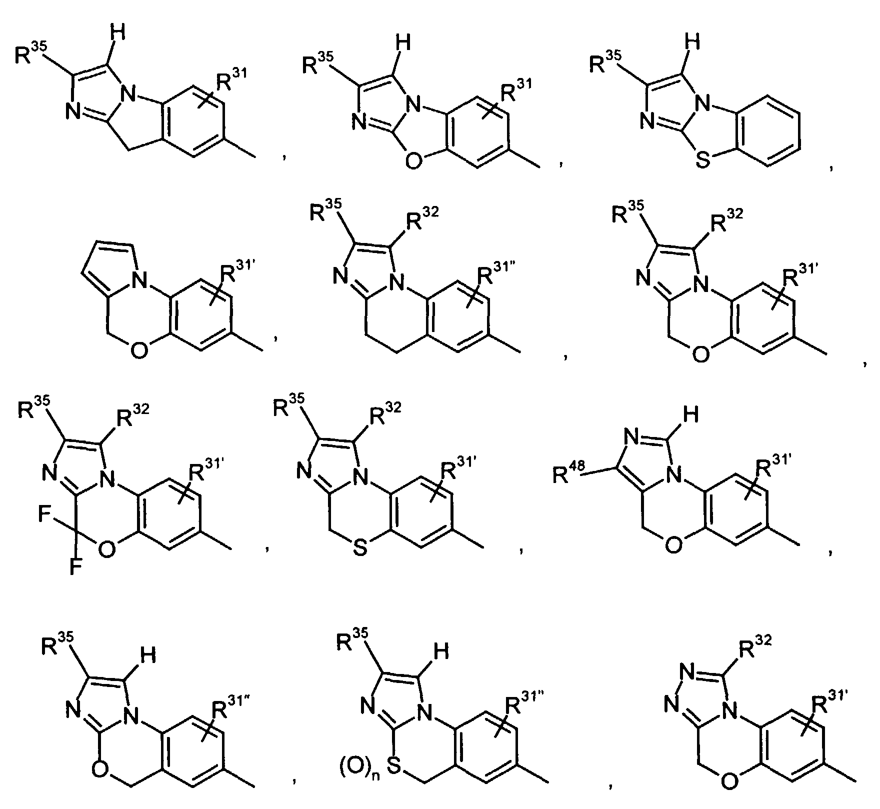 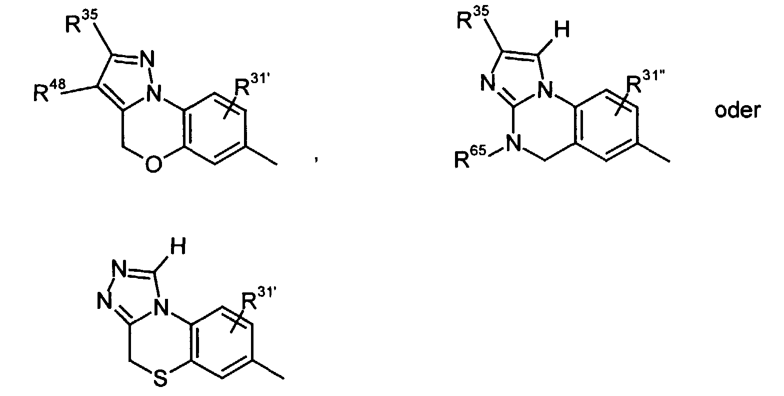 steht,
worin
n eine Zahl 0, 1 oder 2 bedeutet,
R³¹, R^{31'} und R^{31''} gleich oder verschieden sind und Wasserstoff oder Fluor bedeuten,
R³² und R⁴⁸ gleich oder verschieden sind und Wasserstoff oder für Methyl stehen,
R³⁵ für Wasserstoff, Halogen, Cyano, Trifluormethyl, Phenyl oder geradkettiges oder verzweigtes Alkyl, Alkoxycarbonyl oder Alkoxy mit jeweils bis zu 3 Kohlenstoffatomen steht,
R⁶⁵ Wasserstoff oder Methyl bedeutet,
und tautomere Formen und/oder Salze davon.

Bevorzugt sind in einer weiteren Ausführungsform der Erfindung Verbindungen, worin A für einen Rest der Formeln 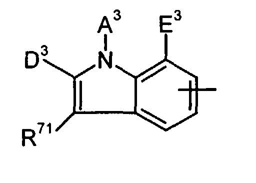 steht, in welcher
A³ und D³ gemeinsam unter Einbezug des Stickstoffatoms einen heterocyclischen Rest der Formel 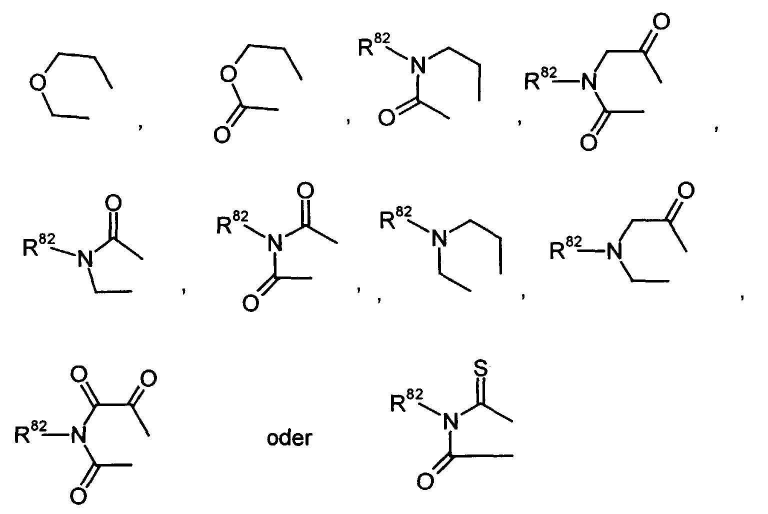 stehen,
   in welcher
   R⁸² Wasserstoff, geradkettiges oder verzweigtes Alkyl mit bis zu 3 Kohlenstoffatomen bedeutet, das gegebenenfalls durch Cyano, Methoxycarbonyl, Ethoxycarbonyl, Amino, N,N-Dimethylamino oder durch Phenyl substituiert ist, oder geradkettiges oder verzweigtes Acyl mit bis zu 3 Kohlenstoffatomen bedeutet, das gegebenenfalls durch Hydroxy, Amino oder N,N-Dimethylamino substituiert ist, oder Methoxycarbonyl bedeutet,
E für Wasserstoff oder Fluor steht,
R⁷¹ für Wasserstoff, Fluor steht,
und tautomere Formen und/oder Salze davon.

Bevorzugt sind in einer weiteren Ausführungsform der Erfindung Verbindungen, worin A für einen Rest der Formeln 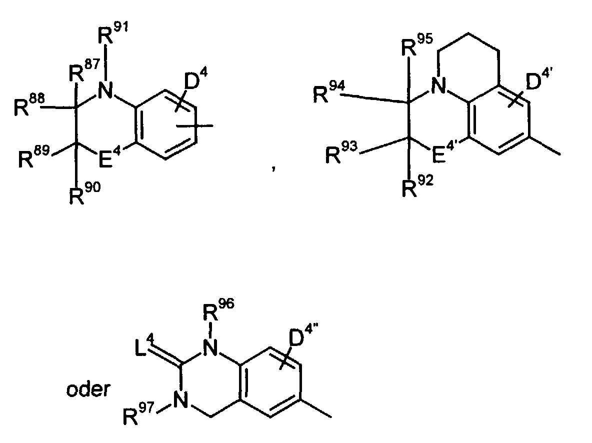 steht,
worin
D⁴, D⁴'und D⁴'' gleich oder verschieden sind und Wasserstoff bedeuten,
E⁴ und E⁴' gleich oder verschieden sind und die -CH₂-Gruppe, ein Sauerstoff-oder Schwefelatom oder einen Rest der Formel -SO oder -SO₂ bedeuten,
L⁴ ein Sauerstoff- oder Schwefelatom oder eine Gruppe der Formel -NR⁹⁸ bedeutet,
   worin
   R⁹⁸ Wasserstoff, Hydroxy oder geradkettiges oder verzweigtes Alkoxy mit bis zu 4 Kohlenstoffatomen bedeutet,
R⁸⁷, R⁸⁸, R⁸⁹, R⁹⁰, R⁹², R⁹³, R⁹⁴ und R⁹⁵ gleich oder verschieden sind und Wasserstoff oder geradkettiges oder verzweigtes Alkyl mit bis zu 3 Kohlenstoffatomen bedeuten, das gegebenenfalls durch Phenyl oder Naphthyl substituiert ist, die ihrerseits ein- bis mehrfach durch Methoxy, Fluor oder Chlor substituiert sein können,
R⁸⁷ und R⁸⁸, R⁸⁹ und R⁹⁰, R⁹² und R⁹³ und/oder R⁹⁴ und R⁹⁵ gemeinsam Gruppen der Formel =O, =CH₂ oder =CHR⁹⁹ bilden,
   worin
   R⁹⁹ Phenyl oder Pyridyl bedeutet, wobei die Ringsysteme gegebenenfalls ein- bis mehrfach durch Fluor, Chlor, oder durch Methoxy substituiert sind,
R⁹¹, R⁹⁶ und R⁹⁷ gleich oder verschieden sind und Wasserstoff, geradkettiges oder verzweigtes Alkyl oder Alkoxycarbonyl mit jeweils bis zu 3 Kohlenstoffatomen bedeuten, oder
   einen Rest der Formel -CO-R¹⁰⁰ bedeuten,
   worin
   R¹⁰⁰ Phenyl, Naphthyl, Pyridyl, Thienyl, Furyl, Imidazolyl, Pyridazolyl, Pyrimidyl oder geradkettiges oder verzweigtes Alkyl mit bis zu 3 Kohlenstoffatomen bedeutet, das gegebenenfalls durch Phenyl oder Naphthyl substituiert ist, wobei die unter R¹⁵ aufgeführten Ringsysteme gegebenfalls ein- bis mehrfach gleich oder verschieden durch Fluor, Chlor, Brom, Trifluormethyl, Nitro, Hydroxy oder durch geradkettiges oder verzweigtes Alkyl oder Alkoxy mit jeweils bis zu 3 Kohlenstoffatomen substituiert sind,
sowie tautomere Formen und/oder Salze davon.

Bevorzugt sind in einer weiteren Ausführungsform der Erfindung Verbindungen, worin A für einen Rest der Formeln 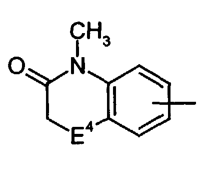 steht, worin
E⁴ ein Sauerstoff- oder Schwefelatom bedeutet, oder die CH₂-Gruppe bedeutet,
und Tautomere und/oder Salze davon.

Bevorzugt sind in einer weiteren Ausführungsform der Erfindung Verbindungen, worin A für einen Rest der Formeln 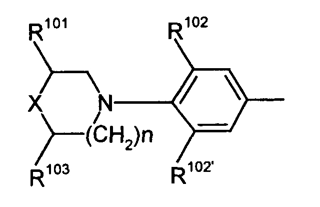 steht,
worin
X O ist;
R¹⁰¹ und R¹⁰³ Wasserstoff ist, und,
R¹⁰² und R^{102'} unabhängig voneinander Wasserstoff, Chlor oder Fluor bedeuten,
R¹⁰³ Wasserstoff ist,
n 1 ist,
und Tautomere und/oder Salze davon.

Bevorzugt sind in einer weiteren Ausführungsform der Erfindung Verbindungen, worin R¹ ausgewählt wird aus Vinyl, Ethinyl, Methyl, Ethyl, n-Propyl, Allyl, Benzyl, Phenyl, Trifluormethyl oder Difluormethyl.

Gegenstand der Erfindung ist weiterhin ein Verfahren zur Herstellung der Verbindungen der allgemeinen Formel (I): 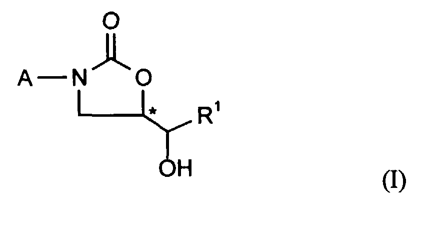 das umfaßt:
[A] die Umsetzung der Verbindungen der allgemeinen Formel (II)

   A-NH₂ (II)

   mit enantiomerenreinen oder racemischen Verbindungen der allgemeinen Formel (III) 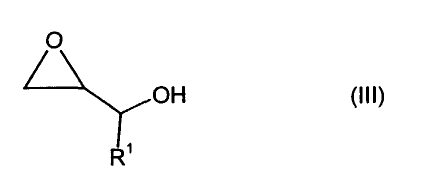 worin A und R¹ die im Anspruch 1 angegebene Bedeutung besitzen, in inerten Lösemitteln und in Anwesenheit eines Hilfsmittels zu enantiomerenreinen oder racemischen Verbindungen der Formel (IV) 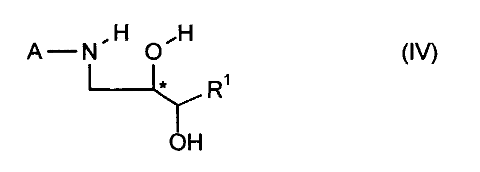 die weiter mit Carbonyl-diimidazol in inerten Lösemitteln zu enantiomerenreinen oder racemischen Verbindungen der allgemeinen Formel (I) cyclisiert werden,
   oder
[B] die Umsetzung von Verbindungen der Formel (V) 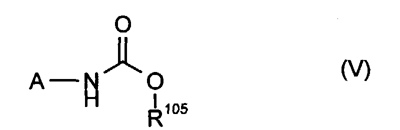 worin A die im Anspruch 1 angegebene Bedeutung aufweist und R¹⁰⁵ wahlweise substituiertes, vorzugsweise durch 1 bis 3 Halogenatome substituiertes (C₁-C₆)Alkyl, wie z.B. Methyl, Ethyl, Propyl oder Trifluormethyl, Phenyl, substituiertes Phenyl, bevorzugt 2- oder 4-Nitrophenyl, oder bevorzugt Benzyl ist,
   mit enantiomerenreinen oder racemischen Verbindungen der allgemeinen Formel (VI) 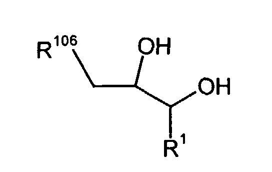 worin R¹ die im Anspruch 1 angegebene Bedeutung aufweist und R¹⁰⁶ eine übliche Abgangsgruppe ist, wie z.B. Halogen, wie Chlor oder Brom, oder -O-SO2-R107, worin R¹⁰⁷ (C₁-C₆)Alkyl, oder gegebenfalls (C₁-C₆)alkylsubstituiertes Phenyl ist, in Gegenwart von Basen zu Verbindungen der allgemeinen Formel (I) und gegebenfalls anschließend die Umwandlung am Rest R¹ in z.B. Ester oder Amide nach üblichen Methoden.

Als hier verwendete Basen eignen sich in Abhängigkeit von den einzelnen Verfahrensschritten für das Verfahren [B] die üblichen anorganischen oder organischen Basen. Hierzu gehören bevorzugt Alkalihydroxide wie beispielsweise Natrium-oder Kaliumhydroxid oder Alkalicarbonate wie Natrium- oder Kaliumcarbonat oder Alkalialkoholate wie beispielsweise Natrium- oder Kaliummethanolat oder Natrium-oder Kaliumethanolat oder Natrium- oder Kalium-tert.-butoxid oder organische Amine wie Ethyldiisopropylamin, Triethylamin, Picolin, Pyridine oder N-Methylpiperidin, oder Amide wie Natriumamid oder Lithiumdiisopropylamid oder Lithium-N-silylalkylamide, wie beispielsweise Lithium-N-(bis)triphenysilylamid oder Lithiumalkyle wie n-Butyllithium.

Basen, wie z.B. Lithium-, oder Natrium- oder Kalium-tert.-amylalkoholat sind besonders bevorzugt für den Schritt [B].

Das erfindungsgemäße Verfahren [A] kann beispielhaft durch folgendes Schema dargestellt werden:

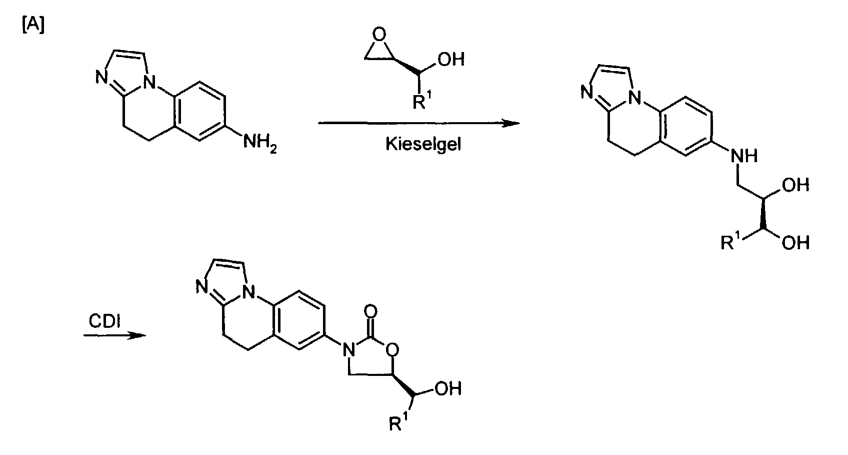

Als Lösemittel für das Verfahren [A] eignen sich die üblichen Lösemittel. Bevorzugt sind Dichlormethan und Chloroform für die Umsetzung mit dem Epoxid und THF für den Ringschluß.

Als Hilfsmittel zur Umsetzung mit dem Epoxid eignen sich schwach saure Katalysatoren, z.B. Kieselgel oder Reaktionsführung unter Druck.

Als Reagenzien für den Ringschluß eignen sich Carbonylverbindungen wie Carbonyldiimidazol, Phosgen, Diphosgen oder Triphosgen, oder Chlorameisensäureester wie Ethyl-, Phenyl-, Nitrophenyl-, Benzyl- oder Isobutyl-chlorformiat, gegebenenfalls in Anwesenheit von Basen wie Triethylamin, Pyridin oder Ethyldiisopropylamin. Bevorzugt ist Carbonyldiimidazol (CDI).

Im allgemeinen wird in einem Temperaturbereich von -78°C bis +50°C, vorzugsweise bei Raumtemperatur, gearbeitet. Beim Ringschluß mit CDI liegt die Reaktionstemperatur zwischen Raumtemperatur und dem Siedepunkt des Tetrahydrofurans.

Das erfindungsgemäße Verfahren [B] kann beispielhaft durch folgendes Schema dargestellt werden: (Für R¹ = H ist ein analoges Verfahren in der WO 97/37980 beschrieben.) 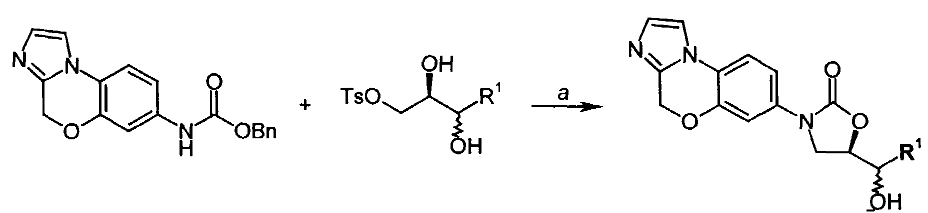
R¹ = z.B. Vinyl, Ethinyl, Me, Et, *n*-Pr, Allyl, Benzyl, Phenyl oder CF₃
Ts = p-Me-Ph-SO₂-

*a: z.B.* 3.1 Äquiv. *tert*.-*A*mylalkohol/2.1 Äquiv. *n*-BuLi, Dimethylacetamid, 0°C/30 min, 22°C/15 h.

Für dieses Verfahren geeignete Lösungsmittel sind z.B.:
Amide wie N,N-Dimethylacetamid, N-Methylpyrrolidin-2-on oder N,N,N',N',N'',N''-Hexamethylphosphorsäuretriamid oder Ether wie Diethylether, Dioxan, Tetrahydrofuran, Glykoldimethylether oder Kohlenwasserstoffe wie Benzol, Toluol, Xylol, Hexan, Cyclohexan oder Erdölfraktionen. Ebenso ist es möglich, Gemische der genannten Lösemittel zu verwenden. Bevorzugt sind N,N-Dimethylacetamid und N-Methylpyrrolidin-2-on.

Die Temperatur der Umsetzung liegt zweckmäßig zwischen -78°C und +80°C. Bevorzugt ist ein Temperaturbereich zwischen -15°C und Raumtemperatur.

Alle Umsetzungen werden im allgemeinen bei normalem, erhöhtem oder bei erniedrigtem Druck durchgeführt (z.B. 0,5 bis 5 bar). Im allgemeinen arbeitet man bei Normaldruck.

Die Darstellung der Verbindungen der allgemeinen Formel (II):

A-NH₂ (II)

erfolgt durch Reduktion der Verbindungen der Formel (VII)

A-NO₂ (VII).

Die Reduktionen können im allgemeinen durch Wasserstoff in Wasser oder in inerten organischen Lösemitteln wie Alkoholen, Ethern oder Halogenkohlenwasserstoffen oder Ammoniumformiat oder deren Gemischen mit Katalysatoren wie Raney-Nickel, Palladium, Palladium auf Tierkohle oder Platin oder mit Hydriden oder Boranen in inerten Lösemitteln, gegebenenfalls in Anwesenheit eines Katalysators, durchgeführt werden.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol oder Ether wie Diethylether, Dioxan, Tetrahydrofuran, Glykoldimethylether oder Diethylenglykoldimethylether oder Amide wie Hexamethylphosphorsäuretriamid oder Dimethylformamid oder Essigsäure. Ebenso ist es möglich, Gemische der genannten Lösemittel zu verwenden. Besonders bevorzugt ist Methanol.

Die Verbindungen der allgemeinen Formel (VII) sind größtenteils bekannt oder als Species neu und können dann im Fall der 4H-Pyrrolo[1,2][1,4]-benzoxazine in Analogie zu bekannten Publikationen M. Kato, Chem. Pharm. Bull. Jpn. 43, 1995, 1358-63, im Fall der substituierten oder unsubstituierten 4H-1,2,4-Triazolo[3,4-c][1,4]-benzoxazinen zu den Publikationen L. Garanti, J, Het. Chem. 13, 1976, 1339-41; B.P. Medaer, Tetrahedron 52, 1996, 8813-26; B.P. Medaer, Tetrahedron 35, 1994, 9767-9776 und im Fall der 4H-Pyrazolo[5,1-c][1,4]benzoxazinen W.-D. Rudorf, J. Prakt. Chem. 329, 1987, 55-61 und 348; im Fall der 4H-Imidazo-[2,1-c][1,4]-benzoxazine in Analogie zu H. Bartsch, J. Het. Chem. 26, 1989, 205-7 hergestellt werden.

Im Fall der 4,5-Dihydro-imidazo[1,2-a]-chinaline werden zunächst die entsprechenden Nitro-3,4-dihydro-1H-chinolin-2-one durch Umsetzung mit Schwefelsäure und Kaliumnitrat bei -15°C in die 2-(2-Dimethoxyethylamino)-nitro-3,4-dihydrochinoline umgesetzt, in einem zweiten Schritt in Analogie zu der Publikation T. Jen, J. Med. Chem. 16, 1973, 633-7 mit Triethyloxonium-tetrafluorborat in Dichlormethan und Aminoacetaldehyd-dimethylacetat und abschließend mit Salzsäure versetzt.

Außerdem können die Verbindungen der Formel (VII) hergestellt werden in Analogie zu Reaktionen, die beschrieben sind in Comprehensive Heterocyclic Chemistry (A.R. Katritzky) Vol. 3, Seiten 995 - 1037 und Vol. 5, Seiten 305-345, 631-639, 660-668, 882-890. Desweiteren sei auf folgende Handbuchserien verwiesen: The Chemistry of Heterocyclic Compounds (A. Weissberger), Progress in Heterocyclic Chemistry (G.W. Gribble) und Advances in Heterocyclic Chemistry (A.R. Katritzky).

Weiterhin sei erwähnt: D. R. Shridhar et al. SYNTHESIS 1982, 986 - 987; Comprehensive Heterocyclic Chemistry Volume 4, 348-372 (1984) und Houben-Weyl, Methoden der Organischen Chemie, Band, E6b2 915 - 1236, (1994)] hergestellt werden.

Im Fall, daß das unter A aufgeführte heterocyclische Ringsystem eine freie N-Funktion trägt, kann diese zunächst nach bekannten Alkylierungsmethoden alkyliert werden.

Die Verbindung der Formel (VIIa) 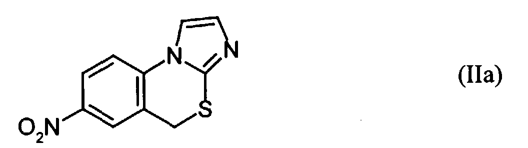 ist neu und kann hergestellt werden, indem man zunächst durch Umsetzung von 2-Amino-5-nitrobenzylalkohol und Thioharnstoff mit HBr unter Rückfluß und anschließender Alkalisch-Stellung 2-Amino-6-nitro-4H-benz-1,3-thiazin herstellt, und abschließend durch Erhitzen mit Chloracetaldehydlösung das Imidazol aufbaut. Der erste Schritt der Umsetzung erfolgt in einem Temperaturbereich von 80 bis 110°C, vorzugsweise bei 100°C. Die Umsetzung mit der Aldehydlösung erfolgt in einem Temperaturbereich von 60°C bis 90°C, vorzugsweise bei 80°C. Die Umsetzung mit Chloracetaldehydlösung erfolgt in Dimethylformamid oder Ethanol in einem Temperaturbereich von 30°C bis 100°C, vorzugsweise bei 70°C. Alle Reaktionsschritte werden bei Normaldruck durchgeführt.

Die Verbindungen der allgemeinen Formel (II) sind größtenteils neu und können wie oben beschrieben durch Reduktion der entsprechenden Nitroverbindungen der allgemeinen Formel (VII) hergestellt werden.

Der Aufbau der Ausgangsverbindungen der allgemeinen Formeln (II) und (VII) kann z.B. auch analog der in der EP-A-0738726 beschriebenen Herstellungsweise erfolgen.

Die Verbindungen der Formel (III) sind an sich bekannt und nach üblichen Verfahren herstellbar.

Die MHK-Werte wurden mit Hilfe der Mikrodilutionsmethode in BH-Medium bestimmt. Jede Prüfsubstanz wurde in DMSO gelöst. In der Mikrotiterplatte wurde durch serielle Verdünnung eine Konzentrationsreihe der Prüfsubstanzen angelegt. Zur Inokulation wurden Übernachtkulturen der Erreger verwandt, die zuvor im Nährmedium 1:250 verdünnt wurden. Zu 100 µl der verdünnten, wirkstoffhaltigen Nährlösungen wurden je 100 µl Inokulationslösung gegeben.

Die Mikrotiterplatten wurden bei 37°C bebrütet und nach ca. 20 Stunden oder nach 3 bis 5 Tagen abgelesen. Der MHK-Wert (µg/ml) gibt die niedrigste Wirkstoffkonzentration an, bei der kein Wachstum zu erkennen war.

### MHK-Werte (µg/ml):

| **Bsp.-Nr.** | **S.aureus 133** |
|---|---|
| 1 (Diastereomer 1 /Diastereomer 2 = 100:1) | 1,5 |
| 2 (Diastereomer 1 /Diastereomer 2 = 100:1) | 1 |

Die erfindungsgemäßen Verbindungen weisen bei geringer Toxizität ein breites antibakterielles Spektrum, speziell gegen gram-positive Keime und einige gram-negative Bakterien sowie Mycobacterien, Corynebakterien, Haemophilus influenzae und anaerobe Keime auf Diese Eigenschaften ermöglichen ihre Verwendung als chemotherapeutische Wirkstoffe in der Human- und Tiermedizin.

Die erfindungsgemäßen Verbindungen sind gegen ein breites Spektrum von Mikroorganismen wirksam. Mit ihrer Hilfe können gram-positive Keime, gram-negative Bakterien und bakterienähnliche Mikroorganismen wie Mycoplasmen bekämpft sowie die durch diese Erreger hervorgerufenen Erkrankungen verbinden, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human-und Tiermedizin geeignet, die durch solche Erreger hervorgerufen werden.

Zur vorliegenden Erfindung gehören pharmazeutische Zubereitungen, die neben nicht-toxischen, inerten, pharmazeutisch geeigneten Trägerstoffen eine oder mehrere erfindungsgemäße Verbindungen enthalten, oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg, Körpergewicht.

Die erfindungsgemäßen Verbindungen können zum Zweck der Erweiterung des Wirkungsspektrums und um eine Wirkungssteigerung zu erreichen, auch mit anderen Antibiotika kombiniert werden.

### Beispiele

### Beispiel 1:

### (5R)-N-(Imidazo[2,1-c]-1,4-benzoxazin-6-yl)-5-(1-hydroxy-2-propenyl)-1,3-oxazolidin-2-on (Diastereomer 1 und Diastereomer 2)

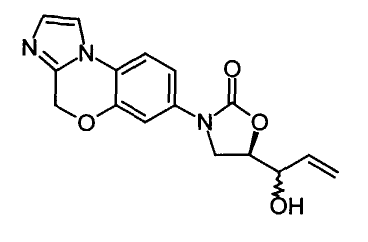

### a) (2R)-1,2-O-Isopropyliden-4-penten-1,2,3-triol

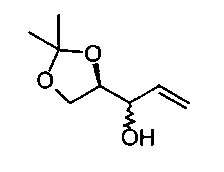

Unter Argon werden bei -10°C 30.0 ml (29.56 mmol) einer 1.0-molaren Lösung von Vinylmagnesiumbromid in THF mit weiteren 10 ml wasserfreiem THF verdünnt und tropfenweise mit einer Lösung von 2.85 g (21.90 mmol) (2R)-2,3-O-Isopropyliden-2,3-hydroxypropanal in 20 ml THF versetzt. Das Kältebad wird entfernt und man läßt das Gemisch auf Raumtemperatur kommen. Nach 1.5 Stunden werden ca. 40 ml gesättigte Ammoniumchlorid-Lösung zugesetzt und das Produkt mit Essigester extrahiert. Die organische Phase wird nacheinander mit gesättigter Natriumhydrogencarbonat-Lösung, Wasser und gesättigter Natriumchlorid-Lösung gewaschen. Nach Trocknen über Natriumsulfat wird das Lösemittel abrotiert. Es werden 2.41 g (15.23 mmol, 70 % Ausbeute) einer farblosen Flüssigkeit erhalten. Laut NMR-Analyse liegt das Produkt als Diastereomerengemisch in einem Verhältnis von 59 zu 41 vor.
R_{f}: 0.52 (Cyclohexan/Essigester, 1:1). ¹H-NMR (300 MHz, CDCl₃, δ/ppm): 5.89-5.74 (1H, m), 5.42-5.22 (2H, m), 4.31-3.77 (4H, m), 2.44 und 2.23 (1H, 2d), 1.46 und 1.38 (6H, 2 s). MS (EI, m/z): 143 (M⁺-CH₃^{·}).

### b) (2R)-4-Penten-1,2,3-triol

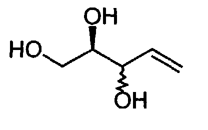

Eine Lösung von 2.30 g (14.54 mmol) (2R)-1,2-O-Isopropyliden-4-penten-1,2,3-triol in 100 ml Methanol wird mit 1 ml konzentrierter Salzsäure versetzt. Nach 15 Stunden bei Raumtemperatur wird Amberlyst A-21 zugesetzt, 30 Minuten gerührt und filtriert. Das Filtrat wird vollständig vom Lösemittel befreit. Der Rückstand wird mit Essigester aufgenommen und auf eine Kieselgelnutsche aufgebracht. Nicht umgesetztes Startmaterial wird mit Essigester herausgewaschen. Das Produkt wird mit Methanol eluiert. Es werden 0.92 g (7.75 mmol, 53 % Ausbeute) eines weissen Feststoffs erhalten.
R_{f}: 0.38 (Cyclohexan/Essigester, 1:1). ¹H-NMR (300 MHz, CDCl₃, δ/ppm): 5.98-5.85 (1H, m), 5.42-5.27 (2H, m), 4.31 und 4.21 (1H, 2 pseudo-quart), 3.81-3.60 (3H, m), 2.79 und 2.71 (1H, 2 d), 2.47 und 2.43 (1H, 2 d), 2.22 und 2.20 (1H, 2 d). MS (EI, m/z): 87 (M⁺-CH₂OH^{·}).

### c) ((2R)-2,3-Hydroxy-4-penten-1-yl)-para-toluolsulfonat

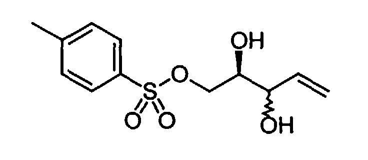

Eine Lösung von 900 mg (7.62 mmol) (2R)-4-Penten-1,2,3-triol in 20 ml wasserfreiem Dichlormethan wird mit 1.85 ml (22.86 mmol) Pyridin versetzt und auf 0°C abgekühlt. Bei dieser Temperatur werden portionsweise insgesamt 1.60 g (8.38 mmol) *para*-Toluolsulfonsäurechlorid zugesetzt. Man läßt über Nacht bei Raumtemperatur rühren. Anschließend wird mit 2-molarer Salzsäure, Wasser und gesättigter Natriumchlorid-Lösung gewaschen. Die organische Phase wird über Natriumsulfat getrocknet. Das gewünschte Produkt wird in Form eines weissen Feststoffs nach Flash-Chromatographie an Kieselgel (Cyclohexan/Essigester, 2:1) erhalten. 920 mg (3.38 mmol, 44 % Ausbeute).
R_{f}: 0.61 (Ethylacetat). ¹H-NMR (300 MHz, CDCl₃, δ/ppm): 7.80 (2H, d), 7.37 (2H, d), 5.91-5.78 (1H, m), 5.37-5.22 (2H, m), 4.23-4.02 (3H, m), 3.88 und 3.79 (1H, 2 pseudo-quint), 2.68 (1H, s broad), 2.47 (3H, s), 2.32 (1H, s). MS (CI, NH₃, m/z): 290 (M+NH₄⁺).

### d) (5R)-N-(Imidazo[2,1-c]-1,4-benzoxazin-6-yl)-5-(1-hydroxy-2-propenyl)-1,3-oxazolidin-2-on (Diastereomer 1 und Diastereomer 2)

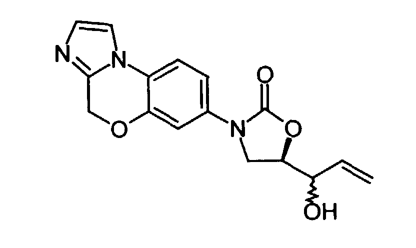

0.62 ml (5.64 mmol) *tert.*-Amylalkohol werden in 6.0 ml Hexan gelöst vorgelegt und unter Argon bei -10°C mit 2.39 ml (3.82 mmol) einer 1.6-molaren Lösung von *n*-Butyllithium in Hexan versetzt. Nach 30 Minuten wird diese Lösung bei 0°C zu einer Lösung von 732 mg (1.82 mmol) Benzyl-N-(imidazo[2,1-c]-1,4-benzoxazin-6-yl)-carbamat in 2.5 ml N,N-Dimethylacetamid gegeben. Nach weiteren 30 Minuten bei 0°C werden 595 mg (2.19 mmol) ((2*R*)-2,3-Hydroxy-4-penten-1-yl)-*para*-toluolsulfonat (V. Jäger et al., Tetrahedron 47, 2195 (1991)) hinzugefügt und das Kältebad entfernt. Das Reaktionsgemisch wird über Nacht bei Raumtemperatur gerührt. Bei 0°C wird das Reaktionsgemisch mit 6 ml Methanol, 6 ml Wasser und 0.4 ml Eisessig versetzt. Es wird mit Dichlormethan verdünnt und nacheinander mit Wasser und gesättigter Natriumchlorid-Lösung gewaschen. Nach Trocknen der organischen Phase über Natriumsulfat wird das Lösemittel abrotiert. Der verbleibende Rückstand wird durch Flash-Chromatographie (Kieselgel, Dichlormethan/Methanol, 100:4) gereinigt. Das Produkt wird in Form eines weissen Feststoffs erhalten. 271 mg (0.86 mmol, 48 % Ausbeute, Diastereomerenverhältnis 71:29).
Das Diastereomerengemisch wird durch präparative HPLC (Kromasil 100, NH-2, 5 µm; *tert*.-Butylmethylether/Methanol, 90:10) in seine Komponenten zerlegt. Es werden 118 mg des Diastereomers 1 und 35 mg des Diastereomers 2 in Form weisser amorpher Feststoffe erhalten.

### Diastereomer 1 (Wirksameres Diastereomer):

Fp.: 227°C. R_{f}: 0.20 (Dichlormethan/Methanol, 100:5). ¹H-NMR (300 MHz, DMSO-d₆, δ/ppm): 7.92 (1H, d), 7.69 (1H, d), 7.48 (1H, d), 7.28 (1H, dd), 7.13 (1H, d), 5.88 (1H, ddd), 5.64 (1H, d), 5.42 (1H, ddd), 5.31 (2H, s), 5.26 (1H, ddd), 4.67 (1H, ddd), 4.32 (1H, pseudo-quart), 4.02 (1H, pseudo-t), 3.82 (1H, dd). MS (CI, m/z): 314 (M+H⁺).

### Diastereomer 2:

Fp.: 187°C. R_{f}: 0.20 (Dichlormethan/Methanol, 100:5). ¹H-NMR (300 MHz, DMSO-d₆, δ/ppm): 7.91 (1H, d), 7.70 (1H, d), 7.44 (1H, d), 7.28 (1H, dd), 7.11 (1H, d), 5.88 (1H, ddd), 5.57 (1H, d), 5.39 (1H, ddd), 5.31 (2H, s), 5.22 (1H, ddd), 4.64 (1H, ddd), 4.20 (1H, pseudo-quart), 4.09 (1H, pseudo-t), 3.87 (1H, dd). MS (CI, m/z): 314 (M+H⁺).

### Beispiel 2:

### (5R)-N-(Imidazo[2,1-c]-1,4-benzoxazin-6-yl)-5-(1-hydroxy-2-propinyl)-1,3-oxazolidin-2-on (Diastereomer 1 und Diastereomer 2)

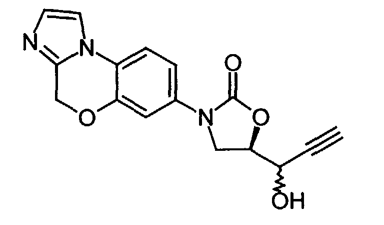

### a) (2R)-1,2-O-Isopropyliden-4-pentin-1,2,3-triol (HAMI 391-1-1)

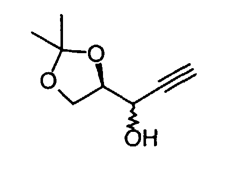

Unter Argon werden bei -10°C 71.5 ml (35.78 mmol) einer 0.5-molaren Lösung von Ethinylmagnesiumbromid in THF mit weiteren 10 ml wasserfreiem THF verdünnt und tropfenweise mit einer Lösung von 3.45 g (26.50 mmol) (2R)-2,3-O-Isopropyliden-2,3-hydroxypropanal in 40 ml THF versetzt. Das Kältebad wird entfernt und man läßt das Gemisch auf Raumtemperatur kommen. Nach 1.5 Stunden werden ca. 40 ml gesättigte Ammoniumchlorid-Lösung zugesetzt und das Produkt mit Essigester extrahiert. Die organische Phase wird nacheinander mit gesättigter Natriumhydrogencarbonat-Lösung, Wasser und gesättigter Natriumchlorid-Lösung gewaschen. Nach Trocknen über Natriumsulfat wird das Lösemittel abrotiert. Es werden 3.45 g (22.06 mmol, 83 % Ausbeute) einer farblosen Flüssigkeit erhalten. Laut GC-und NMR-Analyse liegt das Produkt als Diastereomerengemisch in einem Verhältnis von 55:45 vor.
R_{f}: 0.38 (Cyclohexan/Essigester, 1:1). ¹H-NMR (300 MHz, DMSO-d₆, δ/ppm): 5.72 und 5.69 (1H, 2 d), 4.22 und 4.10 (1H, 2 dt), 4.03-3.97 (2H, m), 3.82-3.74 (1H, m), 3.32 und 3.29 (1H, 2 d), 1.33 und 1.27 (6H, d und s). MS (CI, NH₃, m/z): 174 (M+NH₄⁺), 157 (M+H⁺).

### b) (2R)-4-Pentin-1,2,3-triol

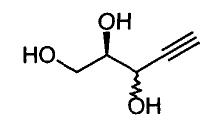

Eine Lösung von 3.23 g (20.66 mmol) (2R)-1,2-O-Isopropyliden-4-pentin-1,2,3-triol in 150 ml Methanol wird mit 1 ml konzentrierter Salzsäure versetzt. Nach 15 Stunden bei Raumtemperatur wird Amberlyst A-21 zugesetzt, 30 Minuten gerührt und filtriert. Das Filtrat wird vollständig vom Lösemittel befreit. Es werden 2.36 g (20.32 mmol, 98 % Ausbeute) eines weissen Feststoffs erhalten.
R_{f}: 0.08 (Dichlormethan/Methanol, 100:10). ¹H-NMR (300 MHz, DMSO-d₆, δ/ppm): 5.32 und 5.28 (1H, 2 d), 4.81 und 4.80 (1H, 2 d), 4.50 und 4.48 (1H 2 d), 4.17-4.11 (1H, m), 3.47-3.33 (3H, m), 3.21 und 3.19 (1H, 2 d). MS (CI, NH₃, m/z): 250 (2·M+NH₄⁺), 134 (M+NH₄⁺).

### c) ((2R)-2,3-Hydroxy-4-pentin-1-yl)-para-toluolsulfonat

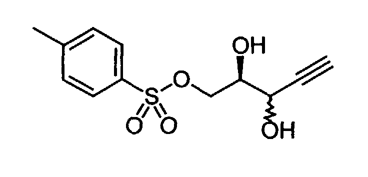

Eine Lösung von 2.32 g (19.95 mmol) (2R)-4-Pentin-1,2,3-triol (R. Brückner et al., Angew. Chem. 108, 1185 (1996). ) in 20 ml wasserfreiem Dichlormethan wird mit 4.84 ml (59.86 mmol) Pyridin versetzt und auf 0°C abgekühlt. Bei dieser Temperatur werden portionsweise insgesamt 4.18 g (21.95 mmol) *para*-Toluolsulfonsäurechlorid zugesetzt. Man läßt über Nacht bei Raumtemperatur rühren. Anschließend wird mit 2-molarer Salzsäure, Wasser und gesättigter Natriumchlorid-Lösung gewaschen. Die organische Phase wird über Natriumsulfat getrocknet. Das gewünschte Produkt wird in Form eines weissen Feststoffs nach Flash-Chromatographie an Kieselgel (Cyclohexan/Essigester, 1:1) erhalten. 1.76 g (6.51 mmol, 31 % Ausbeute).
R_{f}: 0.28 (Dichlormethan/Methanol, 100:5). ¹H-NMR (300 MHz, DMSO-d₆, δ/ppm): 7.78 (2H, d), 7.47 (2H, d), 5.62 (1H, d), 5.49 (1H, d), 4.14-4.07 (2H, m), 3.94-3.86 (1H, m), 3.63-3.58 (1H, m), 3.26 (1H, d), 2.41 (3H, s). MS (CI, NH₃, m/z): 288 (2·M+NH₄⁺), 558 (M+NH₄⁺).

### d) (5R)-N-(Imidazo[2,1-c]-1,4-benzoxazin-6-yl)-5-(1-hydroxy-2-propinyl)-1,3-oxazolidin-2-on (Diastereomer 1 und Diastereomer 2)

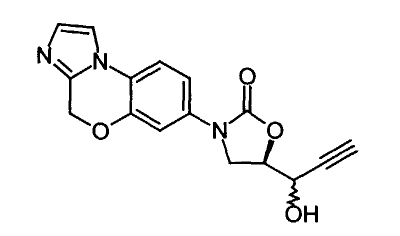

0.81 ml (7.43 mmol) *tert*.-Amylalkohol werden in 7.7 ml Hexan gelöst vorgelegt und unter Argon bei -10°C mit 3.14 ml (5.02 mmol) einer 1.6-molaren Lösung von *n*-Butyllithium in Hexan versetzt. Nach 30 Minuten wird diese Lösung bei 0°C zu einer Lösung von 770 mg (2.40 mmol) Benzyl-N-(imidazo[2,1-c]-1,4-benzoxazin-6-yl)-carbamat in 3.3 ml N,N-Dimethylacetamid gegeben. Nach weiteren 30 Minuten bei 0°C werden 777.3 mg (2.87 mmol) ((2R)-2,3-Hydroxy-4-pentin-1-yl)-*para*-toluolsulfonat hinzugefügt und das Kältebad entfernt. Das Reaktionsgemisch wird über Nacht bei Raumtemperatur gerührt. Bei 0°C wird das Reaktionsgemisch mit 7.7 ml Methanol, 7.7 ml Wasser und 0.6 ml Eisessig versetzt. Es wird mit Dichlormethan verdünnt und nacheinander mit Wasser und gesättigter Natriumchlorid-Lösung gewaschen. Nach Trocknen der organischen Phase über Natriumsulfat wird das Lösemittel abrotiert. Der verbleibende Rückstand wird durch Flash-Chromatographie (Kieselgel, Dichlormethan/Methanol, 100:4) gereinigt. Das Produkt wird in Form eines weissen Feststoffs erhalten. 384 mg (1.23 mmol, 52 % Ausbeute, Diastereomerenverhältnis 52:48).

Das Diastereomerengemisch wird durch präparative HPLC (Kromasil 100, NH-2, 5 µm; *tert*.-Butylmethylether/Methanol, 90:10) in seine Komponenten zerlegt. Es werden 166 mg des Diastereomers 1 und 172 mg des Diastereomers 2 in Form weisser amorpher Feststoffe erhalten.

### Diastereomer 1 (Wirksameres Diastereomer):

Fp.: 238°C (Zers.). R_{f}: 0.07 (Dichlormethan/Methanol, 100:5). ¹H-NMR (300 MHz, DMSO-d₆, δ/ppm): 7.90 (1H, d), 7.69 (1H, d), 7.47 (1H, d), 7.28 (1H, dd), 7.11 (1H, d), 6.13 (1H, d), 5.30 (2H, s), 4.71 (1H, ddd), 4.53 (1H, ddd), 4.18 (1H, pseudo-t), 3.91 (1H, dd), 3.48 (1H, d). MS (CI, NH₃, m/z): 312 (M+H⁺).

### Diastereomer 2:

Fp.: 249°C (Zers.). R_{f}: 0.07 (Dichlormethan/Methanol, 100:5). ¹H-NMR (300 MHz, DMSO-d₆, δ/ppm): 7.90 (1H, d), 7.69 (1H, d), 7.46 (1H, d), 7.27 (1H, dd), 7.12 (1H, d), 6.12 (1H, s broad), 5.31 (2H, s), 4.69 (1H, pseudo-quint), 4.52 (1H, dd), 4.17 (1H, pseudo-t), 3.90 (1H, dd), 3.41 (1H, d). MS (CI, NH₃, m/z): 312 (M+H⁺).

### Beispiel 3

### (5R)-N-(Imidazol[2,1-c]-1,4-benzoxazin-6-yl)-5-(1-hydroxy-ethyl)-1,3-oxazolidin-2-on

Beispiel 3 wurde in der gleichen Weise wie die Oxazolidinone in Beispiel 1 und 2 (siehe oben) hergestellt. Ausbeute: 61 % für den letzten Schritt. Weißer Feststoff. Diastereomerenverhältnis: 67:33.
R_{f}: 0.18 (Dichloromethane/methanol, 100:5). ¹H-NMR (200 MHz, DMSO-d₆, δ/ppm): 7.91 (1H, d), 7.71 (1H, d), 7.48 und 7.47 (1H, dd), 7.13 (1H, d), 5.31 (2H, s), 5.23 und 5.14 (1H, 2 d), 4.58-4.43 (1H, m), 4.12-3.71 (3H, m), 1.15 und 1.10 (3H, 2 d). MS (El, m/z): 301 (M⁺).

### Beispiel 4

### (5R)-N-(Imidazol[2,1-c]-1,4-benzoxazin-6-yl)-5-(1-hydroxy-propyl)-1,3-oxazolidin-2-on

Beispiel 4 wurde in der gleichen Weise wie die Oxazolidinone in Beispiel 1 und 2 (siehe oben) hergestellt. Ausbeute: 25 % für den letzten Schritt. Weißer Feststoff Diastereomerenverhältnis: 50:50.
R_{f}: 0.25 (Dichloromethane/Methanol, 100:5). ¹H-NMR (300 MHz, DMSO-d₆, δ/ppm): 7.90 (1H, d), 7.70 (1H, d), 7.47 und 7.44 (1H, 2 d), 7.29 (1H, dd), 7.11 (1H, d), 5.31 (2H, s), 5.21 und 5.09 (1H, 2 d), 4.62-4.51 (1H, m), 4.07 (1H, pseudo-quart), 3.93-3.34 (1H, m), 3.12-3.57 (1H, m), 1.52-1.28 (2H, m), 0.95 (3H, t). MS (El, m/z): 315 (M⁺).

### Beispiel 5

### (5R)-N-(Imidazo[2,1-c]-1,4-benzoxazin-6-yl)-5-(1-hydroxy-butyl)-1,3-oxazolidin-2-on

Beispiel 5 wurde in der gleichen Weise wie die Oxazolidinone in Beispiel 1 und 2 (siehe oben) hergestellt. Ausbeute: 62 % für den letzten Schritt. Weisser Feststoff. Diastereomerenverhältnis: 55:45.
R_{f}: 0.26 (Dichloromethane/Methanol, 100:5). ¹H-NMR (300 MHz, DMSO-d₆, δ/ppm): 7.91 (1H, d), 7.70 (1H, d), 7.48 und 7.45 (1H, 2 d), 7.30 und 7.29 (1H, 2 dd), 7.11 (1H, d), 5.31 (2H, s) 5.22 und 5.10 (1H, 2 d), 4.59-4.48 (1H, m), 4.10-4.01 (1H, m), 3.92 und 3.37 (1H, 2 dd), 3.72-3.68 und 3.57-3.51 (1H, 2 m), 1.52-1.40 (2H, m), 1.40-1.30 (2H, m), 0.91 (3H, t). MS (Cl, NH₃, m/z): 330 (M+H⁺).

### Beispiel 6

### (5R)-N-(Imidazo[2,1-c]-1,4-benzoxazin-6-yl)-5-(1-hydroxy-3-butenyl)-1,3-oxazolidin-2-on

Beispiel 6 wurde in der gleichen Weise wie die Oxazolidinone in Beispiel 1 und 2 (siehe oben) hergestellt. Ausbeute: 47 % für den letzten Schritt. Weisser Feststoff. Diastereomerenverhältnis: 63:37.
R_{f}: 0.22 (Dichloromethane/Methanol, 100:5). ¹H-NMR (300 MHz, DMSO-d₆, δ/ppm): 7.92 (1H, d), 7.71 (1H, d), 7.50 und 7.47 (1H, 2 d), 7.33-7.29 (1H, m), 7.13 (1H, d), 5.97-5.82 (1H, m), 5.33 (2H, s), 5.39 und 5.28 (1H, 2 d), 5.18-5.07 (2H, m), 4.66-4.50 (1H, m), 4.13-3.78 (2H, m), 3.84-3.78 und 3.69-3.62 (1H, 2 m), 2.32-2.14 (2H, m). MS (Cl, NH₃ m/z): 328 (M+H⁺).

### Beispiel 7

### (5R)-N-(Imidazo[2,1-c]-1,4-benzoxazin-6-yl)-5-(1-hydroxy-2-phenyl-ethyl)-1,3-oxazolidin-2-on

Beispiel 7 wurde in der gleichen Weise wie die Osazolidinone in Beispiel 1 und 2 (siehe oben) hergestellt. Ausbeute: 24 % für den letzten Schritt. Weisser Feststoff. Diastereomerenverhältnis: 43:57.
R_{f}: 0.16 (Dichloromethane/Methanol, 100:5). ¹H-NMR (200 MHz, DMSO-d₆, δ/ppm): 7.91 (1H, d), 7.71 und 7.70 (1H, 2 d), 7.52-7.41 (2H, m), 7.32-7.18 (5H, m), 7.12 (1H, d), 5.83 und 5.77 (1H, d und s), 5.31 (2H, s), 4.98-4.83 (1H, m) 4.60-3.50 (1H, m), 4.10 und 4.09 (1H, 2 pseudo-t), 3.90 (1H, dd), 2.83-2.80 und 2.33 (2H, m und s). MS (Cl, NH₃, m/z): 395 (M+NH₄H⁺), 378 (M+H⁺).

### Beispiel 8

### (5R)-N-(Imidazol[2,1-c]-1,4-benzoxazin-6-yl)-5-(1-hydroxy-1-phenyl-methyl)-1,3-oxazolidin-2-on

Beispiel 8 wurde in der gleichen Weise wie die Oxazolidinone in Beispiel 1 und 2 (siehe oben) hergestellt. Ausbeute: 41 % für den letzten Schritt. Weisser Feststoff. Diastereomerenverhältnis: 50:50.
R_{f}: 0.11 (Dichloromethane/Methanol, 100:5). ¹H-NMR (300 MHz, DMSO-d₆, δ/ppm, Einzeldiastereomer): 7.88 (1H, d), 7.66 (1H, 2 d), 7.44 (2H, d), 7.36 (2H, t), 7.29 (2H, d), 7.20 (1H, dd), 7.11 (1H, d), 5.91 (1H, d), 5.29 (2H, s), 4.88-4.78 (2H, m), 4.07 (1H, pseudo-t), 3.88 (1H, dd. MS (Cl, NH₃, m/z): 364 (M+H⁺).

### Beispiel 9

### (5R)-N-(Imidazol[2,1-c]-1,4-benzoxazin-6-yl)-5-(1-hydroxy-2,2,2-trifluorethyl)-1,3-oxazolidin-2-on

### a) (2R)-4,4,4-Trifluor-1,2,3-butantriol

Unter Argon werden 4,82 g (33,88 mmol) Trifluormethyltrimethylsilan zu einer Lösung von 3,50 g (26,89 mmol) (2R)-2,3-O-Isopropylidenglyceraldehyd in 60 ml trockenem THF gegeben. Die Mischung wird auf 0°C abgekühlt und 60 mg Tetra-n-butylammoniumfluoridtrihydrat werden zugegeben. Nach 10 Minuten wird das Kältebad entfernt und das Reaktionsgemisch 3 Stunden bei Raumtemperatur gerührt. Dann werden 6 ml 2-molare Salzsäure zugegeben und das Gemisch wird 15 Stunden weitergerührt. Der Ionenaustauscher Amberlyst A-21 wird hinzugefügt und nach 30-minütigem Rühren durch Filtration entfernt. Das Filtrat wird eingeengt, bis es vollkommen trocken ist. Das Produkt wird durch Saugfiltration über einer Schicht Kieselerde mit Essigester als Elutionsmittel gereinigt. 3,1 g (19,26 mmol, Ausbeute: 72 %) eines weissen Feststoffes werden als Distereomerengemisch im Verhältnis 56:44 erhalten.
R_{f}: 0.48 (Essigester). ¹H-NMR (300 MHz, DMSO-d₆, δ/ppm): 6.17 und 5.80 (1H, 2 d), 4.91 und 4.78 (1H, 2 d), 4.71 und 4.52 (1H, 2 t), 4.06-3,75 (1H, m), 3.69-3.33 (3H, m). MS (Cl, NH₃, m/z): 195 (m+NH₃+NH₄⁺).

### b) (2R)-4,4,4-Trifluor-2,3-dihydroxy-1-para-toluolsulfonat

Bei 0°C werden 3,93 g (20,61 mmol) para-Toluolsulfonylchlorid portionsweise in eine Lösung von 3,0 g (18,74 mmol) (2R)-4,4,4-Trifluor-1,2,3-butantriol und 3,03 ml (37,45 mmol) Pyridin in 50 ml trockenem Dichlormethan gegeben. Das Reaktionsgemisch läßt man langsam auf Raumtemperatur erwärmen und über Nacht stehen. Wasser wird hinzugefügt und die organische Schicht wird mit einer Salzlösung gewaschen und über Natriumsulfat getrocknet. Das Produkt wird durch Flash-Chromatographie (Kieselgel, Cyclohexan/Essigester, 3:1) isoliert. 1,80 g (5,73 mmol, Ausbeute: 31 %) eines weissen Feststoffes werden erhalten.
R_{f}: 0.20 (Cyclohexane/Essigester, 2:1). ¹H-NMR (300 MHz, DMSO-d₆, δ/ppm): 7.80 (2H, d), 7.50 (2H, d), 6.51 und 6.22 (1H; 2 d), 5.65 und 5.49 (1H, 2 d), 4.10-3.78 (4H, m), 2.43 (3H, s). MS (Cl, NH₃, m/z): 3.49 (M+NH₃+NH₄⁺), 332 (M+NH₄⁺).

### c) (5R, 1'?)-N-(Imidazo[2,1-c]-1,4-benzoxazin-6-yl)-5-(1-hydroxy-2,2,2-trifluorethyl)-1,3-oxazolidin-2-on

180 ml (1,54 mmol) tert.-Amylalkohol werden in 150 ml trockenem Hexan gelöst. Unter Argon werden 700 ml (1,05 mmol) einer 1,6-molaren Lösung von n-Butyllithium in Hexan bei -10°C zugegeben. Nach 30 Minuten wird diese Lösung von Lithium tert.-Amylat bei 0°C zu einer Lösung von 200 mg (0,50 mmol) Benzyl-N-(imidazo[2,1-c]-1,4-benzoxazin-6-yl)-carbamat in 700 ml N,N-Dimethylacetamid gegeben. Nach weiteren 30 Minuten bie 0°C werden 187,8 mg (0,60 mmol) (2R)-4,4,4-Trifluor-2,3-dihydroxy-1-paratoluolsulfonat hinzugefügt und das Kältebad entfernt. Das Reaktionsgemisch wird über 15 Stunden bei Raumtemperatur gerührt. Dann wird es wieder auf 0°C abgekühlt und mit 1,5 ml Methanol, 1,5 ml Wasser und 0,1 ml Essigsäure versetzt. Das Produkt wird mit Dichlormethan extrahiert und die organische Schicht nacheinander mit Wasser und einer Salzlösung gewaschen und über Natriumsulfat getrocknet. Nach Filtration und Verdampfung des Lösemittels wird das Produkt durch Flash-Chromatographie (Kieselgel, Dichlormethan/Methanol, 100:5 isoliert. Zwei Fraktionen werden erhalten.

### Fraktion 1:

42,4 mg (119,3 µmol, 24 %) Ausbeute: hellgelber Feststoff, Einzelisomer Fp.: 271°C (Zers.). R_{f}: 0.18 (Dichloromethan/Methanol, 100:5). ¹H-NMR (300 MHz, DMSO-d₆, δ/ppm): 7.90 (1H, s), 7.71 (1H, d), 7.42 (1H, d), 7.28 (1H, dd), 7.12 (1H, s), 6.89 (1H, d), 5.30 (2H, s), 4.97 (1H, dd), 4.32 (1H, pseudo-quint), 4.22 (1H, pseudo-t), 3.88 (1H, dd). MS (Cl, NH₃, m/z): 373 (M+NH₄⁺), 356 (M+H⁺).

### Fraktion 2:

39,0 mg (118,4 µmol, 24 %) Ausbeute: weniger Farbstoff, Einzelisomer; Fp.: 189°C, R_{f}: 0.12 (Dichloromethan/Methanol, 100:5). ¹H-NMR (300 MHz, DMSO-d₆, δ/ppm): 7.72 (1H, d), 7.39 (1H, d), 7.03 (1H, d), 6.37 (1H, s), 6.36 (1H, d), 6.00 (1H, d), 5.83 (1H, t), 5.17 (2H, s), 5.08 (1H, d), 4.03-3.93 (1H, m), 3.88-3.82 (1H, m), 3.22 (1H, pseudo-t), 3.03 (1H, dd). MS (Cl, NH₃ m/z): 347 (M+NH₄⁺), 330 (M+H⁺).

## Patentansprüche

1. Verbindungen der allgemeinen Formel (I) 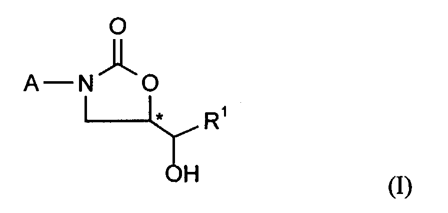 in welcher
R¹ geradkettiges oder verzweigtes Alkyl mit bis zu 6 Kohlenstoffatomen ist, das gegebenfalls mit 1 bis 3 Substituenten substituiert ist, die aus der Gruppe ausgewählt werden, die besteht aus Halogen, Cyano, Hydroxy, geradkettigem oder verzweigtem Alkoxy mit 2 bis 6 Kohlenstoffatomen, Mercapto, geradkettigem oder verzweigtem Alkylthio mit 2 bis 6 Kohlenstoffatomen, Aryl mit 6 bis 10 Kohlenstoffatomen und -NR²R³, worin
R² und R³ Wasserstoff bedeuten, oder
R² Wasserstoff bedeutet, und
R³ einen Rest der Formeln 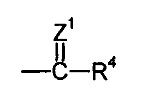 bedeutet,
worin
Z¹ ein Sauerstoff- oder Schwefelatom bedeutet,
R⁴ geradkettiges oder verzweigtes Alkoxy mit bis zu 8 Kohlenstoffatomen oder Trifluormethyl bedeutet, oder
Cycloalkyl mit 3 bis 6 Kohlenstoffatomen bedeutet, das gegebenenfalls durch Halogen oder Aryl mit 6 bis 10 Kohlenstoffatomen substituiert ist, oder
Aryl mit 6 bis 10 Kohlenstoffatomen oder einen 5- bis 6-gliedrigen gesättigten oder aromatischen Heterocylcus mit bis zu 3 Heteroatomen aus der Reihe S, N und/oder O bedeutet, wobei die unter R⁴ aufgeführten Ringsysteme gegebenenfalls bis zu 2-fach gleich oder verschieden durch Halogen, Cyano, Nitro, Hydroxy oder Phenyl substituiert sind,
oder
R⁴ geradkettiges oder verzweigtes Alkyl mit bis zu 6 Kohlenstoffatomen bedeutet, das gegebenenfalls durch Phenoxy, Benzyloxy, Carboxyl, Halogen oder geradkettiges oder verzweigtes Alkoxycarbonyl oder Acyl mit jeweils bis zu 6 Kohlenstoffatomen oder durch einen 5-bis 6-gliedrigen Heterocyclus aus der Reihe S, N und/oder O substituiert ist,
oder
R⁴ einen Rest der Formel -NR⁵R⁶ bedeutet, worin
R⁵ und R⁶ gleich oder verschieden sind und Wasserstoff, Phenyl, Pyridyl oder geradkettiges oder verzweigtes Alkyl mit bis zu 5 Kohlenstoffatomen bedeuten, das gegebenenfalls durch über N-gebundenes Morpholin substituiert ist, oder
R¹ geradkettiges oder verzweigtes Alkenyl mit 2 bis 6 Kohlenstoffatomen; geradkettiges oder verzweigtes Alkinyl mit 2 bis 6 Kohlenstoffatomen; geradkettiges oder verzweigtes Alkoxycarbonyl mit bis zu 6 Kohlenstoffatomen; Aryl mit 6 bis 10 Kohlenstoffatomen oder -CONR² 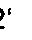 R³ 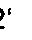 ist, worin R² 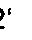 und R³ 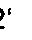 die oben angegebene Bedeutungen von R² und R³ aufweisen, oder
A für einen Rest der Formeln 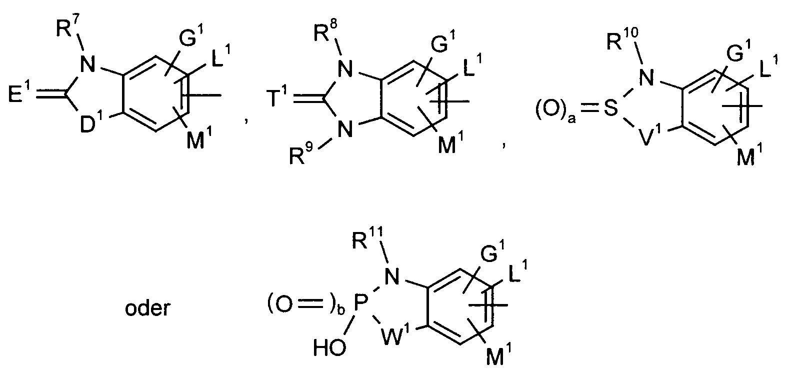 steht,
worin
G¹, L¹ und M¹ gleich oder verschieden sind und für Wasserstoff, Carboxy, Halogen, Cyano, Formyl, Trifluormethyl, Nitro, für geradkettiges oder verzweigtes Alkyl mit bis zu 6 Kohlenstoffatomen oder für eine Gruppe der Formel -CO-NR¹²R¹³ stehen,
worin
R¹² und R¹³ gleich oder verschieden sind und Wasserstoff, geradkettiges oder verzweigtes Alkyl mit bis zu 4 Kohlenstoffatomen oder Phenyl bedeuten,
R⁷ Wasserstoff, Cycloalkylcarbonyl oder Cycloalkyl mit jeweils 3 bis 6 Kohlenstoffatomen, oder geradkettiges oder verzweigtes Alkoxycarbonyl mit bis zu 6 Kohlenstoffatomen, oder geradkettiges oder verzweigtes Alkyl oder Alkenyl mit jeweils bis zu 10 Kohlenstoffatomen bedeutet, das gegebenenfalls durch Cyano, Azido, Trifluormethyl, Pyridyl, Halogen, Hydroxy, Carboxyl, geradkettiges oder verzweigtes Alkoxycarbonyl mit bis zu 6 Kohlenstoffatomen, Aryl mit 6 bis 10 Kohlenstoffatomen, Cycloalkyl mit 3 bis 6 Kohlenstoffatomen und/oder durch eine Gruppe der Formel -(CO)_{c}-NR¹⁴R¹⁵, R¹⁶-N-SO₂-R¹⁷, R¹⁸R¹⁹-N-SO₂-, R²⁰-S(O)_{d} oder 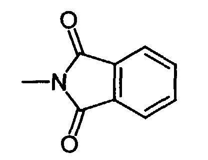 substituiert ist,
worin
c eine Zahl 0 oder 1 bedeutet,
R¹⁴, R¹⁵, R¹⁶, R¹⁸ und R¹⁹, die oben angegebene Bedeutung von R¹² und R¹³ haben und mit dieser gleich oder verschieden sind,
oder gemeinsam mit dem Stickstoffatom einen 5- bis 6-gliedrigen, gesättigten Heterocyclus mit gegebenenfalls einem weiteren Heteroatom aus der Serie N, S und/oder O bilden, der seinerseits gegebenenfalls, auch an einem weiteren Stickstoffatom, durch geradkettiges oder verzweigtes Alkyl oder Acyl mit bis zu 3 Kohlenstoffatomen substituiert sein kann,
d eine Zahl 0, 1 oder 2 bedeutet,
R¹⁷ und R²⁰ gleich oder verschieden sind und geradkettiges oder verzweigtes Alkyl mit bis zu 4 Kohlenstoffatomen, Benzyl, Phenyl oder Tolyl bedeuten,
oder R⁷ einen Rest der Formeln 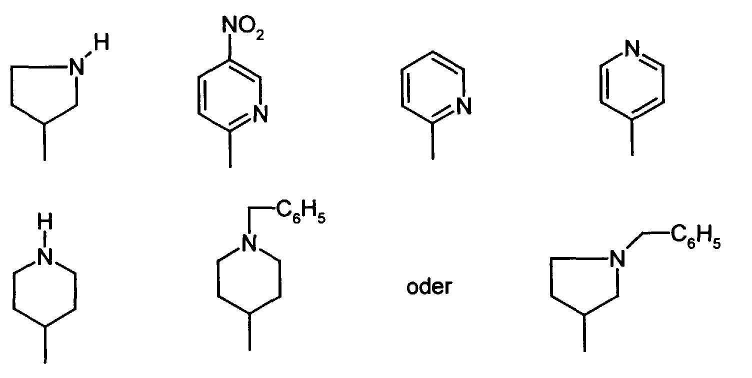 bedeutet oder
R⁷ eine Gruppe der Formel -COCl₃ oder geradkettiges oder verzweigtes Acyl mit bis zu 6 Kohlenstoffatomen bedeutet, das gegebenenfalls durch -CF₃, -CCl₃ oder eine Gruppe der Formel -OR²¹ substituiert ist,
worin
R²¹ Wasserstoff oder geradkettiges oder verzweigtes Alkyl mit bis zu 6 Kohlenstoffatomen bedeutet, das gegebenenfalls durch Aryl mit bis zu 10 Kohlenstoffatomen substituiert ist,
oder
R⁷ eine Gruppe der Formel -(CO)ₑ-NR²²R²³, -NR²⁴-SO₂R²⁵, R²⁷R²⁶N-SO₂ oder R²⁸-S(O)_{f} bedeutet,
worin
e die oben angegebene Bedeutung von c hat und mit dieser gleich oder verschieden ist,
R²², R²³ und R²⁴ die oben angegebene Bedeutung von R¹⁴, R¹⁵ und R¹⁶ haben und mit dieser gleich oder verschieden sind,
R²⁶ und R²⁷ die oben angegebene Bedeutung von R¹² und R¹³ haben und mit dieser gleich oder verschieden sind,
f die oben angegebene Bedeutung von d hat und mit dieser gleich oder verschieden ist,
R²⁵ und R²⁸ die oben angegebene Bedeutungen von R¹⁷ und R¹⁹ haben und mit dieser gleich oder verschieden sind,
D¹ ein Sauerstoff oder Schwefelatom bedeutet,
E¹ ein Sauerstoff- oder Schwefelatom oder eine Gruppe der Formel NH bedeutet,
T¹ ein Sauerstoffatom oder die NH-Gruppe bedeutet,
R⁸ und R⁹ die oben angegebene Bedeutung von R⁷ haben und mit dieser gleich oder verschieden sind,
oder
T¹ ein Schwefelatom bedeutet,
mit der Maßgabe, daß R⁸ und R⁹ die oben angegebene Bedeutung von R⁷ haben, aber nicht für Wasserstoff stehen,
oder im Fall, daß R⁷, R⁸ und R⁹ nicht für Wasserstoff stehen, E¹ und/oder T¹ eine Gruppe der Formel NR²⁹ bedeuten, worin R²⁹ mit Ausnahme von Wasserstoff die oben angegebene Bedeutung von R⁷ hat und mit dieser gleich oder verschieden ist,
oder R²⁹ Cyano oder eine Gruppe der Formel -CO₂R³⁰ bedeutet, worin R³⁰ Benzyl oder Phenyl bedeutet, die gegebenenfalls durch Nitro oder Halogen substituiert sind
V¹ und W¹ die oben angegebene Bedeutung von D¹ haben oder die oben aufgeführte Gruppe N-R⁹ bedeuten und mit dieser gleich oder verschieden sind,
a eine Zahl 1 oder 2 bedeutet,
b eine Zahl 0 oder 1 bedeutet,
R¹⁰ und R¹¹ die oben angegebene Bedeutung von R⁷ haben und mit dieser gleich oder verschieden sind, oder
A für Reste der Formeln 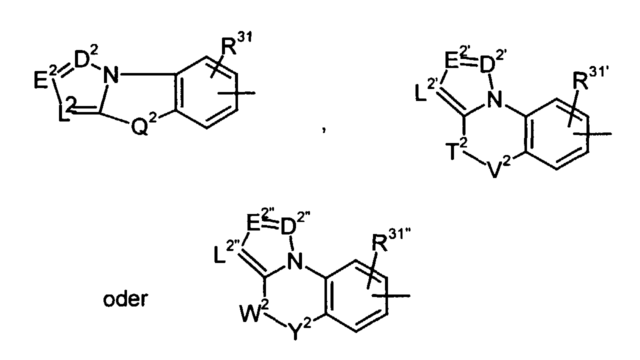 steht,
worin
R³¹, R^{31'} und R^{31''} gleich oder verschieden sind und Wasserstoff oder Halogen bedeuten,
D², D²' und D²'' gleich oder verschieden sind und ein Stickstoffatom oder einen Rest der Formel CR³² bedeuten,
worin
R³² Wasserstoff, Trifluormethyl, Halogen, Hydroxy, geradkettiges oder verzweigtes Alkoxy mit bis zu 6 Kohlenstoffatomen oder einen Rest der Formel -NR³³R³⁴ bedeutet,
worin
R³³ und R³⁴ gleich oder verschieden sind und Wasserstoff, Aryl mit 6 bis 10 Kohlenstoffatomen, Benzyl oder geradkettiges oder verzweigtes Alkyl oder Acyl mit jeweils bis zu 6 Kohlenstoffatomen bedeuten,
E², E²' und E²'' gleich oder verschieden sind und ein Stickstoffatom oder einen Rest der Formel CR³⁵ bedeuten,
worin
R³⁵ Wasserstoff, Trifluormethyl, Nitro, Cyano oder Halogen bedeutet, oder
geradkettiges oder verzweigtes Alkyl mit bis zu 6 Kohlenstoffatomen oder Benzyl bedeutet, die gegebenenfalls durch Hydroxy oder durch geradkettiges oder verzweigtes Alkoxy mit bis zu 4 Kohlenstoffatomen substituiert sind, oder
Aryl mit 6 bis 10 Kohlenstoffatomen oder einen aromatischen Heterocyclus mit bis zu 3 Heteroatomen aus der Reihe S, N und/oder O bedeutet, wobei die Ringsysteme gegebenenfalls bis zu 3-fach gleich oder verschieden durch Halogen, Hydroxy, Nitro, geradkettiges oder verzweigtes Alkyl mit bis zu 4 Kohlenstoffatomen oder Trifluormethyl substituiert sind, oder
R³⁵ Reste der Formeln O-R³⁶, -CO-R³⁷ oder -NR³⁸R³⁹ bedeutet,
worin
R³⁶ Wasserstoff, Benzoyl, geradkettiges oder verzweigtes Alkyl oder Acyl mit jeweils bis zu 6 Kohlenstoffatomen, Benzyl oder Aryl mit 6 bis 10 Kohlenstoffatomen oder einen aromatischen Heterocyclus mit bis zu 3 Heteroatomen aus der Reihe S, N und/oder O bedeutet,
R³⁷ Hydroxy, geradkettiges oder verzweigtes Alkyl oder Alkoxy mit jeweils bis zu 6 Kohlenstoffatomen, Benzyl oder Aryl mit 6 bis 10 Kohlenstoffatomen oder einen aromatischen Heterocyclus mit bis zu 3 Heteroatomen aus der Reihe S, N und/oder O bedeutet,
oder
R³⁷ eine Gruppe der Formel -NR⁴⁰R⁴¹ bedeutet,
worin
R⁴⁰ und R⁴¹ gleich oder verschieden sind und Wasserstoff, Aryl mit 6 bis 10 Kohlenstoffatomen, Benzyl oder geradkettiges oder verzweigtes Alkyl mit bis zu 6 Kohlenstoffatomen bedeuten,
R³⁸ und R³⁹ gleich oder verschieden sind und Wasserstoff, Benzyl, Aryl mit 6 bis 10 Kohlenstoffatomen, geradkettiges oder verzweigtes Alkyl mit bis zu 6 Kohlenstoffatomen oder eine Gruppe der Formel -CO₂R⁴² oder -CM²-NR⁴³R⁴⁴ bedeuten,
worin
R⁴² geradkettiges oder verzweigtes Alkyl mit bis zu 6 Kohlenstoffatomen, Benzyl oder Phenyl bedeutet,
M² ein Sauerstoff- oder Schwefelatom bedeutet,
R⁴³ und R⁴⁴ gleich oder verschieden sind und die oben angegebene Bedeutung von R³³ und R³⁴ haben,
oder
R³⁸ Wasserstoff bedeutet
und
R³⁹ einen Rest der Formel 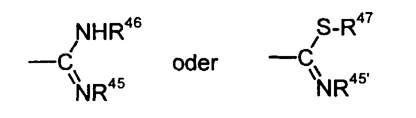 bedeutet,
worin
R⁴⁵ und R^{45'} gleich oder verschieden sind und Wasserstoff, geradkettiges oder verzweigtes Alkyl mit bis zu 6 Kohlenstoffatomen, Aryl mit 6 bis 10 Kohlenstoffatomen oder Benzyl bedeuten,
R⁴⁶ und R⁴⁷ gleich oder verschieden sind und geradkettiges oder verzweigtes Alkyl mit bis zu 6 Kohlenstoffatomen, Phenyl oder Benzyl bedeuten,
L², L²' und L²'' gleich oder verschieden sind und ein Stickstoffatom oder einen Rest der Formel CR⁴⁸ bedeuten,
worin
R⁴⁸ Wasserstoff, Trifluormethyl, Nitro, Cyano, Halogen oder geradkettiges oder verzweigtes Alkyl mit bis zu 6 Kohlenstoffatomen bedeutet, das gegebenenfalls durch Phenyl oder durch einen Rest der Formel -OR⁴⁹ substituiert ist,
worin
R⁴⁹ Wasserstoff, geradkettiges oder verzweigtes Alkyl mit bis zu 6 Kohlenstoffatomen, Aryl mit 6 bis 10 Kohlenstoffatomen oder Benzyl bedeutet,
oder
R⁴⁸ Reste der Formeln -OR⁵⁰, -COR⁵¹ oder -NR⁵²R⁵³ bedeutet,
worin
R⁵⁰ Wasserstoff, Aryl mit 6 bis 10 Kohlenstoffatomen, Benzyl oder geradkettiges oder verzweigtes Alkyl oder Acyl mit jeweils bis zu 6 Kohlenstoffatomen bedeutet,
R⁵¹ die oben angegebene Bedeutung von R³⁹ hat und mit dieser gleich oder verschieden ist,
R⁵² und R⁵³ die oben angegebene Bedeutung von R³³ und R³⁴ haben und mit diesen gleich oder verschieden sind,
oder
R⁵² Wasserstoff bedeutet
und
R⁵³ Cyano oder einen Rest der Formel -CO-NR⁵⁴R⁵⁵ oder -CS-NR⁵⁶R⁵⁷ bedeutet,
worin
R⁵⁴, R⁵⁵, R⁵⁶ und R⁵⁷ gleich oder verschieden sind und die oben angegebene Bedeutung von R³⁵ und R³⁶ haben,
oder
R⁵² und R⁵³ gemeinsam mit dem Stickstoffatom einen 5- bis 6-gliedrigen, gesättigten Heterocyclus bilden, der noch ein weiteres Heteroatom aus der Reihe S, O oder einen Rest der Formel -NH enthalten kann,
Q² ein Sauerstoff- oder Schwefelatom oder Reste der Formeln SO₂, SO, C=O oder CR⁵⁸R⁵⁹ bedeutet,
worin
R⁵⁸ und R⁵⁹ gleich oder verschieden sind und Wasserstoff oder Halogen bedeuten,
T² einen Rest der Formel CR⁶⁰R⁶¹ bedeutet,
worin
R⁶⁰ und R⁶¹ gleich oder verschieden sind und Wasserstoff, Halogen, Hydroxy, geradkettiges oder verzweigtes Alkyl oder Alkoxy mit jeweils bis zu 6 Kohlenstoffatomen oder Benzyloxy bedeuten,
oder
R⁶⁰ und R⁶¹ gemeinsam Reste der Formeln =O, =S, 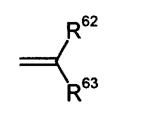 oder =N-R⁶⁴ bilden,
worin
R⁶² und R⁶³ gleich oder verschieden sind und Wasserstoff, geradkettiges oder verzweigtes Alkyl mit bis zu 6 Kohlenstoffatomen oder Benzyl bedeuten,
oder
R⁶² und R⁶³ gemeinsam einen 3- bis 6-gliedrigen, gesättigten oder partiell ungesättigten Carbocyclus bilden,
und
R⁶⁴ Wasserstoff, Benzyl oder geradkettiges oder verzweigtes Alkyl oder Acyl mit jeweils bis zu 6 Kohlenstoffatomen bedeutet,
V² ein Sauerstoffatom, ein Schwefelatom oder einen Rest der Formel SO oder SO₂ bedeutet,
W² ein Sauerstoff- oder Schwefelatom bedeutet, oder Reste der Formeln C=O, C=S, SO, SO₂, NR⁶⁵ oder CR⁶⁶R⁶⁷ bedeutet,
worin
R⁶⁵ die oben angegebene Bedeutung von R⁶⁴ hat und mit dieser gleich oder verschieden ist,
R⁶⁶ und R⁶⁷ gleich oder verschieden sind und Wasserstoff, Halogen, geradkettiges oder verzweigtes Alkyl mit bis zu 6 Kohlenstoffatomen oder Benzyl bedeuten,
oder
R⁶⁶ Wasserstoff bedeutet
und
R⁶⁷ einen Rest der Formel -OR⁶⁸ bedeutet,
worin
R⁶⁸ Wasserstoff, geradkettiges oder verzweigtes Alkyl oder Acyl mit jeweils bis zu 6 Kohlenstoffatomen oder Benzyl bedeutet,
Y² einen Rest der Formel C=O oder -CR⁶⁹R⁷⁰ bedeutet,
worin
R⁶⁹ und R⁷⁰ gleich oder verschieden sind und Wasserstoff, Halogen, Benzyl oder geradkettiges oder verzweigtes Alkyl mit bis zu 6 Kohlenstoffatomen bedeuten,
oder
R⁶⁹ Wasserstoff bedeutet
und
R⁷⁰ Hydroxy, Benzyloxy oder geradkettiges oder verzweigtes Alkoxy mit bis zu 6 Kohlenstoffatomen bedeutet,
oder
W² und Y gemeinsam für die Gruppe -CH=CH- stehen, oder
A für einen Rest der Formel 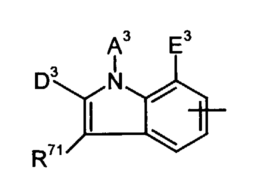 steht, in welcher
R⁷¹ für Wasserstoff, Halogen oder für geradkettiges oder verzweigtes Alkyl mit bis zu 4 Kohlenstoffatomen steht,
E³ für Wasserstoff oder für Halogen steht,
A³ und D³ gemeinsam unter Einbezug des Stickstoffatoms einen heterocyclischen Rest der Formel 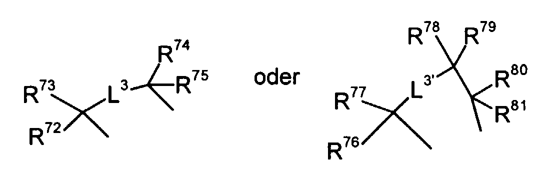 bilden,
worin
L³ und L³' gleich oder verschieden sind und ein Sauerstoffatom oder einen Rest der Formel -NR⁸² bedeuten,
worin
R⁸² Wasserstoff, Carboxyl, Cycloalkyl mit 3 bis 6 Kohlenstoffatomen, geradkettiges oder verzweigtes Alkoxycarbonyl mit bis zu 6 Kohlenstoffatomen bedeutet, oder
geradkettiges oder verzweigtes Acyl mit bis zu 6 Kohlenstoffatomen bedeutet, das gegebenenfalls durch Halogen, Hydroxy, geradkettiges oder verzweigtes Alkoxy mit bis zu 5 Kohlenstoffatomen oder durch eine Gruppe der Formel -NR⁸³R⁸⁴ substituiert ist,
worin
R⁸³ und R⁸⁴ gleich oder verschieden sind und Wasserstoff, Phenyl oder geradkettiges oder verzweigtes Alkyl mit bis zu 5 Kohlenstoffatomen bedeuten, oder
geradkettiges oder verzweigtes Alkyl oder Alkenyl mit jeweils bis zu 6 Kohlenstoffatomen bedeutet, die gegebenenfalls durch Cyano, Halogen, Carboxyl, geradkettiges oder verzweigtes Alkoxycarbonyl mit bis zu 5 Kohlenstoffatomen oder durch eine Gruppe der Formel -NR^{83'}R^{84'} substituiert sind,
worin
R^{83'} und R^{84'} die oben angegebene Bedeutung von R⁸³ und R⁸⁴ haben und mit dieser gleich oder verschieden sind,
und/oder Alkyl oder Alkenyl gegebenenfalls durch Aryl mit 6 bis 14 Kohlenstoffatomen substituiert sind, das seinerseits durch Halogen oder durch geradkettiges oder verzweigtes Alkyl, Alkoxy oder Acyl mit jeweils bis zu 6 Kohlenstoffatomen substituiert sein kann,
R⁷², R⁷³, R⁷⁴ und R⁷⁵ gleich oder verschieden sind und Wasserstoff oder geradkettiges oder verzweigtes Alkyl mit bis zu 6 Kohlenstoffatomen bedeuten, das gegebenenfalls durch Halogen substituiert ist,
oder
R⁷² und R⁷³ und/oder R⁷⁴ und R⁷⁵ gemeinsam Reste der Formel =O, oder =S bilden,
R⁷⁶, R⁷⁷, R⁷⁸, R⁷⁹, R⁸⁰ und R⁸¹ gleich oder verschieden sind und Wasserstoff oder geradkettiges oder verzweigtes Alkyl mit bis zu 6 Kohlenstoffatomen bedeuten, das gegebenenfalls durch Hydroxy, Halogen, geradkettiges oder verzweigtes Alkoxy oder Alkoxycarbonyl mit jeweils bis zu 6 Kohlenstoffatomen, Aryl mit 6 bis 10 Kohlenstoffatomen oder durch eine Gruppe der Formel -NR⁸⁵R⁸⁶ substituiert ist,
worin
R⁸⁵ und R⁸⁶ die oben angegebene Bedeutung von R⁸³ und R⁸⁴ haben und mit dieser gleich oder verschieden sind,
oder
R⁷⁶ und R⁷⁷ und/oder R⁷⁸ und R⁷⁹ und/oder R⁸⁰ und R⁸¹ gemeinsam Reste der Formel =O oder =S bilden
und/oder
R⁷⁹ und R⁸⁰ gemeinsam eine endocyclische Doppelbindung bilden, oder
A für Reste der Formeln 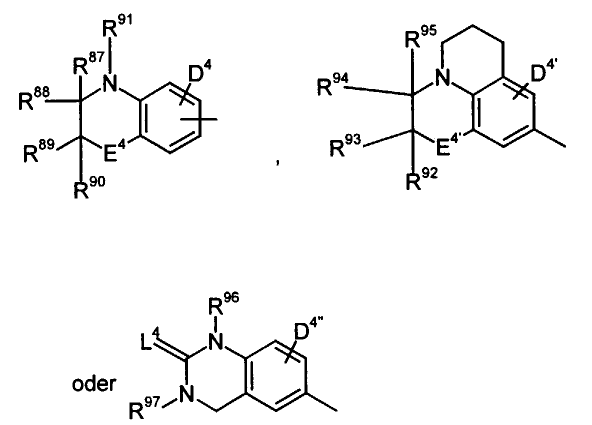 steht,
steht worin
D⁴, D⁴' und D⁴'' gleich oder verschieden sind und Wasserstoff, Carboxy, Halogen, Cyano, Formyl, Trifluormethyl, Nitro, geradkettiges oder verzweigtes Alkoxy, Alkoxycarbonyl, Alkylthio oder Acyl mit jeweils bis zu 6 Kohlenstoffatomen oder geradkettiges oder verzweigtes Alkyl mit bis zu 6 Kohlenstoffatomen bedeuten,
E⁴ und E⁴' gleich oder verschieden sind und eine -CH₂-Gruppe, ein Sauerstoff- oder Schwefelatom oder einen Rest der Formel -SO oder -SO₂ bedeuten,
L⁴ ein Sauerstoff- oder Schwefelatom oder eine Gruppe der Formel =NR⁹⁸ bedeutet,
worin
R⁹⁸ Wasserstoff, Phenyl, Hydroxy oder geradkettiges oder verzweigtes Alkoxy mit bis zu 6 Kohlenstoffatomen bedeutet,
R⁸⁷, R⁸⁸, R⁸⁹, R⁹⁰, R⁹², R⁹³, R⁹⁴ und R⁹⁵ gleich oder verschieden sind und Wasserstoff oder geradkettiges oder verzweigtes Alkyl oder Alkenyl mit jeweils bis zu 6 Kohlenstoffatomen bedeuten, die gegebenenfalls durch Aryl mit 6 bis 10 Kohlenstoffatomen oder durch einen 5- bis 6-gliedrigen aromatischen Heterocyclus mit bis zu 3 Heteroatomen aus der Reihe S, N und/oder O substituiert sind, die ihrerseits ein- bis mehrfach durch geradkettiges oder verzweigtes Alkoxy oder Alkyl mit jeweils bis zu 6 Kohlenstoffatomen, Hydroxy oder Halogen substituiert sein können,
oder
R⁸⁷ und R⁸⁸, R⁸⁹ und R⁹⁰, R⁹² und R⁹³ und/oder R⁹⁴ und R⁹⁵ gemeinsam Gruppen der Formel =O, =CH₂ oder =CHR⁹⁹ bilden,
worin
R⁹⁹ Aryl mit 6 bis 10 Kohlenstoffatomen oder einen 5- bis 7-gliedrigen aromatischen Heterocyclus mit bis zu 3 Heteroatomen aus der Reihe S, N und/oder O bedeutet, wobei die Ringsysteme gegebenenfalls ein- bis mehrfach durch Halogen, Hydroxy oder durch geradkettiges oder verzweigtes Alkyl oder Alkoxy mit jeweils bis zu 6 Kohlenstoffatomen substituiert sind,
R⁹¹, R⁹⁶ und R⁹⁷ gleich oder verschieden sind und Wasserstoff, geradkettiges oder verzweigtes Alkyl oder Alkoxycarbonyl mit jeweils bis zu 6 Kohlenstoffatomen bedeuten, oder
einen Rest der Formel -CO-R¹⁰⁰ bedeuten,
worin
R¹⁰⁰ Aryl mit 6 bis 10 Kohlenstoffatomen, ein 5- bis 7-gliedriger aromatischer Heterocyclus mit bis zu 3 Heteroatomen aus der Reihe S, N und/oder O oder geradkettiges oder verzweigtes Alkyl mit bis zu 4 Kohlenstoffatomen bedeutet, das gegebenenfalls durch Aryl mit 6 bis 10 Kohlenstoffatomen substituiert ist, wobei die unter R¹⁰⁰ aufgeführten Ringsysteme gegebenfalls einbis mehrfach, gleich oder verschieden durch Halogen, Trifluormethyl, Nitro, Hydroxy oder durch geradkettiges oder verzweigtes Alkyl oder Alkoxy mit jeweils bis zu 6 Kohlenstoffatomen substituiert sind, oder
A eine Gruppe der Formel 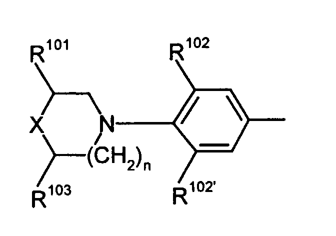
worin X O, S, SO, SO₂, S(O)NR¹⁰⁴ oder S(O)₂NR¹⁰⁴ ist;
worin R¹⁰⁴ Wasserstoff, wahlweise durch ein bis drei Substituenten ausgewählt aus Chlor, Fluor, Hydroxy, (C₁-C₆)Alkoxy, Amino und Mono- oder Di(C₁-C₆)-Alkylamino substituiertes (C₁-C₄)Alkyl oder p-Toluolsulfonyl ist,
R¹⁰¹ Wasserstoff ist, und,
wenn X O ist, R¹⁰¹ Wasserstoff -CH₃, -CN, -CO₂H, -CO₂R¹⁰⁵, worin
R¹⁰⁵ Wasserstoff, wahlweise durch ein bis drei Substituenten ausgewählt aus Chlor, Fluor, Hydroxy, (C₁-C₆)Alkoxy, (C₁-C₆)Acyloxy und -OCH₂-Ph substituiertes (C₁-C₄)Alkyl, (C₃-C₆)Cycloalkyl, Amino, Mono- oder Di(C₁-C₆)Alkylamino oder (C₁-C₆)Alkoxy ist, oder -(CH₂)₉R¹⁰⁶ ist, worin g 1 oder 2 und R¹⁰⁶ Wasserstoff, -OR¹⁰⁵, -OCOR¹⁰⁵, -NH₂, -NHCOR¹⁰⁵ oder -N(R¹⁰⁴)₂ ist, worin R¹⁰⁵ und R¹⁰⁵, die oben angebenen Bedeutungen aufweisen, und
R¹⁰² und R^{102'} unabhängig voneinander Wasserstoff, Chlor oder Fluor bedeuten,
R¹⁰³ Wasserstoff oder Methyl ist,
n 0, 1 oder 2 ist
und tautomere Formen und/oder Salze davon.

2. Verbindungen nach Anspruch 1, worin A für einen Rest der Formeln 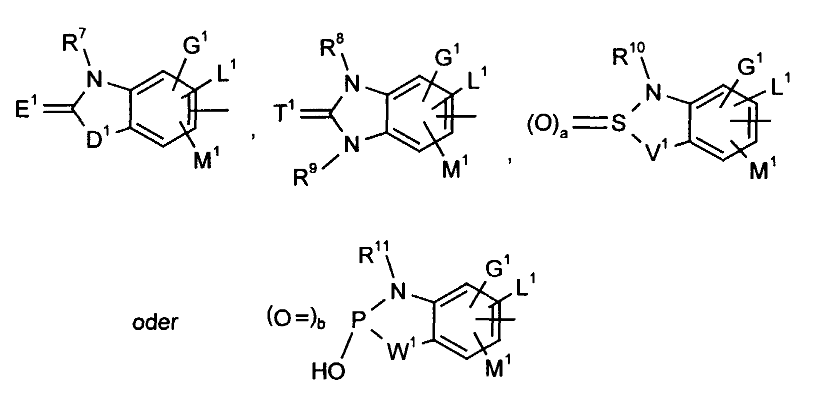 steht,
worin G¹, L¹ und M¹ für Wasserstoff stehen,
R⁷ Wasserstoff, Cyclopropylcarbonyl, Cyclopropyl, Cyclobutyl, Cyclopentyl, Cyclohexyl oder geradkettiges oder verzweigtes Alkoxycarbonyl mit bis zu 3 Kohlenstoffatomen bedeutet oder geradkettiges oder verzweigtes Alkyl oder Alkenyl mit bis zu 8 Kohlenstoffatomen bedeutet, die gegebenenfalls durch Cyano, Azido, Trifluormethyl, Pyridyl, Fluor, Chlor, Brom, Pyridyl, Hydroxy, Carboxyl, geradkettiges oder verzweigtes Alkoxycarbonyl mit bis zu 4 Kohlenstoffatomen, Phenyl, Benzyloxycarbonyl, Cyclopropyl, Cyclopentyl, Cyclohexyl, und/oder durch eine Gruppe der Formel -(CO)_{c}-NR¹⁴R¹⁵, R¹⁶-N-SO₂-R¹⁷, R¹⁸R¹⁹-N-SO₂-, R²⁰-S(O)_{d} oder 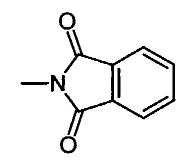 substituiert ist,
c eine Zahl 0 oder 1 bedeutet,
worin R¹⁴, R¹⁵, R¹⁶, R¹⁸ und R¹⁹ gleich oder verschieden sind und Wasserstoff, Methyl oder Ethyl bedeuten,
d eine Zahl 0, 1 oder 2 bedeutet,
R¹⁷ und R²⁰ gleich oder verschieden sind und geradkettiges oder verzweigtes Alkyl mit bis zu 4 Kohlenstoffatomen, Benzyl, Phenyl oder Tolyl bedeuten,
oder R⁷ einen Rest der Formeln 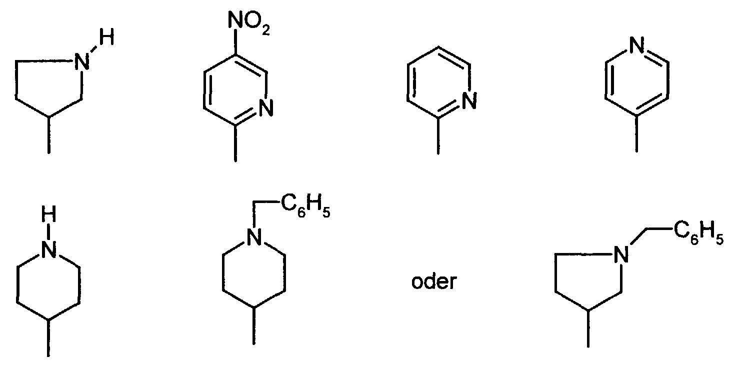 bedeutet oder
R⁷ eine Gruppe der Formel -COCl₃ oder geradkettiges oder verzweigtes Acyl mit bis zu 4 Kohlenstoffatomen bedeutet, das gegebenenfalls durch -CF₃, -CCl₃ oder eine Gruppe der Formel -OR²¹ substituiert ist,
worin
R²¹ Wasserstoff oder geradkettiges oder verzweigtes Alkyl mit bis zu 4 Kohlenstoffatomen bedeutet, das gegebenenfalls durch Phenyl substituiert ist,
oder
R⁷ eine Gruppe der Formel -(CO)ₑ-NR²²R²³ oder R²⁸-S(O)_{f} bedeutet,
worin
e die Zahl 1 ist,
R²² und R²³ gleich oder verschieden sind und Wasserstoff, Methyl oder Ethyl bedeuten,
f die oben angegebene Bedeutung von d hat und mit dieser gleich oder verschieden ist,
R²⁸ Methyl, Phenyl, Tolyl oder Benzyl bedeutet,
D¹ ein Sauerstoff oder Schwefelatom bedeutet,
E¹ ein Sauerstoff- oder Schwefelatom oder eine Gruppe der Formel NH bedeutet,
T¹ ein Sauerstoffatom oder die NH-Gruppe bedeutet,
R⁸ und R⁹ die oben angegebene Bedeutung von R⁷ haben und mit dieser gleich oder verschieden sind,
oder
T¹ ein Schwefelatom bedeutet,
mit der Maßgabe, daß R⁸ und R⁹ die oben angegebene Bedeutung von R⁷ haben, aber nicht für Wasserstoff stehen,
oder im Fall, daß R⁷, R⁸ und R⁹ nicht für Wasserstoff stehen, E¹ und/oder T¹ eine Gruppe der Formel NR²⁹ bedeuten, worin R²⁹ mit Ausnahme von Wasserstoff die oben angegebene Bedeutung von R⁷ hat und mit dieser gleich oder verschieden ist,
oder R²⁹ Cyano oder eine Gruppe der Formel -CO₂R³⁰ bedeutet, worin
R³⁰ Benzyl oder Phenyl bedeutet, die gegebenenfalls durch Nitro oder Halogen substituiert sind;
V¹ und W¹ die oben angegebene Bedeutung von D¹ haben oder die oben aufgeführte Gruppe N-R⁹ bedeuten und mit dieser gleich oder verschieden sind,
a eine Zahl 1 oder 2 bedeutet,
b eine Zahl 0 oder 1 bedeutet,
R¹⁰ und R¹¹ die oben angegebene Bedeutung von R⁷ haben und mit dieser gleich oder verschieden sind,
und tautomere Formen und/oder Salze davon.

3. Verbindungen der allgemeinen Formel (I) nach Anspruch 1, dadurch gekennzeichnet, daß A ausgewählt wird aus der Gruppe der Formeln: 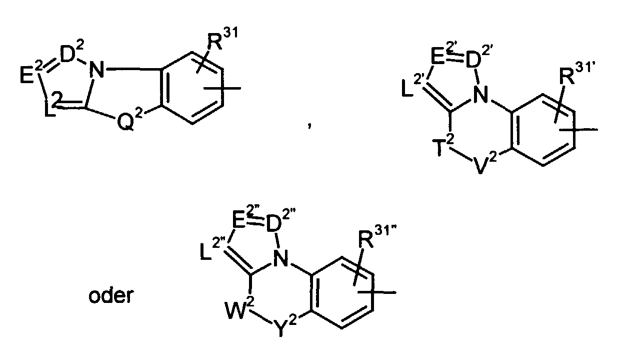 steht,
worin
R³¹, R^{31'} und R^{31''} gleich oder verschieden sind und Wasserstoff oder Fluor bedeuten,
D², D²' und D²'' gleich oder verschieden sind und ein Stickstoffatom oder einen Rest der Formel CR³² bedeuten,
worin
R³² Wasserstoff, Trifluormethyl, Chlor, Fluor, Hydroxy, geradkettiges oder verzweigtes Alkoxy mit bis zu 3 Kohlenstoffatomen bedeutet,
E², E²' und E²'' gleich oder verschieden sind und ein Stickstoffatom oder einen Rest der Formel CR³⁵ bedeuten,
worin
R³⁵ Wasserstoff, Trifluormethyl, Nitro, Cyano, Fluor, Chlor, geradkettiges oder verzweigtes Alkyl mit bis zu 3 Kohlenstoffatomen oder Benzyl bedeutet, die gegebenenfalls durch Hydroxy substituiert sind, oder Phenyl, Naphtyl, Pyridyl, Pyrimidyl, Pyrazinyl, Thienyl oder Furyl bedeutet, oder
R³⁵ Reste der Formeln O-R³⁶, -CO-R³⁷ oder -NR³⁸R³⁹ bedeutet,
worin
R³⁶ Wasserstoff, geradkettiges oder verzweigtes Alkyl oder Acyl mit jeweils bis zu 4 Kohlenstoffatomen, Benzyl oder Phenyl bedeutet,
R³⁷ Hydroxy, geradkettiges oder verzweigtes Alkyl oder Alkoxy mit jeweils bis zu 3 Kohlenstoffatomen, Benzyl oder Phenyl bedeutet,
oder
R³⁷ eine Gruppe der Formel -NR⁴⁰R⁴¹ bedeutet,
worin
R⁴⁰ und R⁴¹ gleich oder verschieden sind und Wasserstoff, Phenyl, Benzyl oder geradkettiges oder verzweigtes Alkyl mit bis zu 3 Kohlenstoffatomen bedeuten,
R³⁸ und R³⁹ gleich oder verschieden sind und Wasserstoff, Benzyl, Phenyl, geradkettiges oder verzweigtes Alkyl mit bis zu 3 Kohlenstoffatomen oder eine Gruppe der Formel -CO₂R⁴² bedeuten,
worin
R⁴² geradkettiges oder verzweigtes Alkyl mit bis zu 3 Kohlenstoffatomen, Benzyl oder Phenyl bedeutet,
L², L²' und L²'' gleich oder verschieden sind und ein Stickstoffatom oder einen Rest der Formel CR⁴⁸ bedeuten,
worin
R⁴⁸ Wasserstoff, Trifluormethyl, Nitro, Cyano, Fluor, Chlor oder geradkettiges oder verzweigtes Alkyl mit bis zu 3 Kohlenstoffatomen bedeutet, das gegebenenfalls durch Phenyl oder durch einen Rest der Formel -OR⁴⁹ substituiert ist,
worin
R⁴⁹ Wasserstoff, geradkettiges oder verzweigtes Alkyl mit bis zu 6 Kohlenstoffatomen, Aryl mit 6 bis 10 Kohlenstoffatomen oder Benzyl bedeutet,
oder
R⁴⁸ Reste der Formeln -OR⁵⁰, -COR⁵¹ oder -NR⁵²R⁵³ bedeutet,
worin
R⁵⁰ Wasserstoff, Phenyl, Benzyl oder geradkettiges oder verzweigtes Alkyl oder Acyl mit jeweils bis zu 3 Kohlenstoffatomen bedeutet,
R⁵¹ die oben angegebene Bedeutung von R³⁹ hat und mit dieser gleich oder verschieden ist,
R⁵² und R⁵³ die oben angegebene Bedeutung von R³³ und R³⁴ haben und mit diesen gleich oder verschieden sind,
Q² ein Sauerstoff- oder Schwefelatom oder Reste der Formeln SO₂, SO, C=O oder CR⁵⁸R⁵⁹ bedeutet,
worin
R⁵⁸ und R⁵⁹ gleich oder verschieden sind und Wasserstoff oder Fluor bedeuten,
T² einen Rest der Formel -CR⁶⁰R⁶¹ bedeutet,
worin
R⁶⁰ und R⁶¹ gleich oder verschieden sind und Wasserstoff, Fluor, Hydroxy, geradkettiges oder verzweigtes Alkyl oder Alkoxy mit jeweils bis zu 3 Kohlenstoffatomen oder Benzyloxy bedeuten,
oder
R⁶⁰ und R⁶¹ gemeinsam Reste der Formeln =O oder =S bilden,
V² ein Sauerstoffatom, ein Schwefelatom oder einen Rest der Formel SO oder SO₂ bedeutet,
W² ein Sauerstoff- oder Schwefelatom bedeutet, oder Reste der Formeln C=O, C=S, SO, SO₂, NR⁶⁵ oder CR⁶⁶R⁶⁷ bedeutet,
worin
R⁶⁵ Wasserstoff, Benzyl oder geradkettiges oder verzweigtes Alkyl oder Acyl mit jeweils bis zu 3 Kohlenstoffatomen bedeutet,
R⁶⁶ und R⁶⁷ gleich oder verschieden sind und Wasserstoff, Fluor, geradkettiges oder verzweigtes Alkyl mit bis zu 3 Kohlenstoffatomen oder Benzyl bedeuten, oder
Y² einen Rest der Formel C=O oder -CR⁶⁹R⁷⁰ bedeutet,
worin
R⁶⁹ und R⁷⁰ gleich oder verschieden sind und Wasserstoff, Fluor, Benzyl oder geradkettiges oder verzweigtes Alkyl mit bis zu 6 Kohlenstoffatomen bedeuten,
und tautomere Formen und/oder Salze davon.

4. Verbindungen der allgemeinen Formel (I) nach Anspruch 3, worin
A für Reste der Formeln 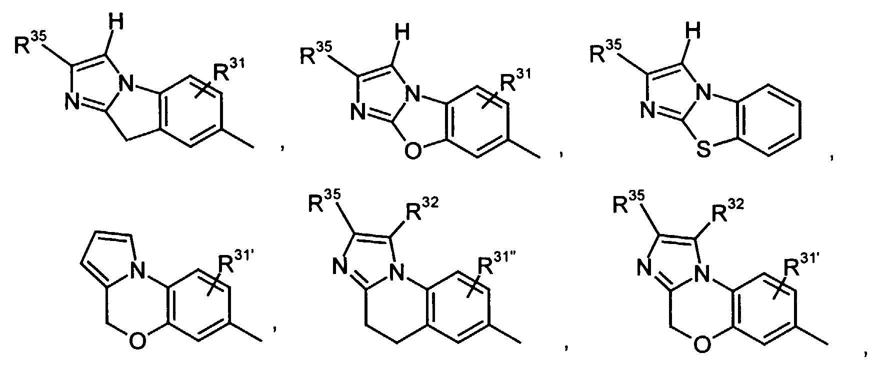 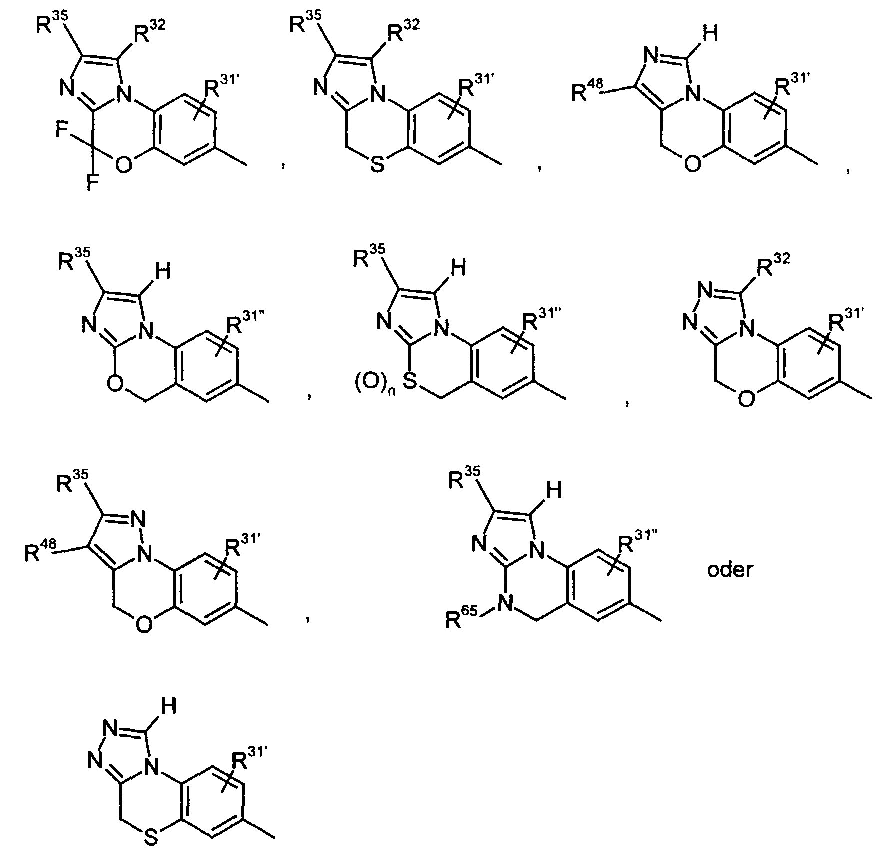 steht,
worin
n eine Zahl 0, 1 oder 2 bedeutet,
R³¹, R^{31'} und R^{31''} gleich oder verschieden sind und Wasserstoff oder Fluor bedeuten,
R³² und R⁴⁸ gleich oder verschieden sind und Wasserstoff oder für Methyl stehen,
R³⁵ für Wasserstoff, Halogen, Cyano, Trifluormethyl, Phenyl oder geradkettiges oder verzweigtes Alkyl, Alkoxycarbonyl oder Alkoxy mit jeweils bis zu 3 Kohlenstoffatomen steht,
R⁶⁵ Wasserstoff oder Methyl bedeutet,
und tautomere Formen und/oder Salze davon.

5. Verbindungen der allgemeinen Formel (I) nach Anspruch 1, worin A für einen Rest der Formel 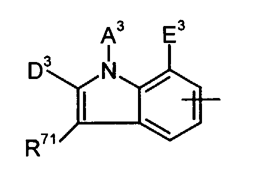 steht, in welcher
A³ und D³ gemeinsam unter Einbezug des Stickstoffatoms einen heterocyclischen Rest der Formel 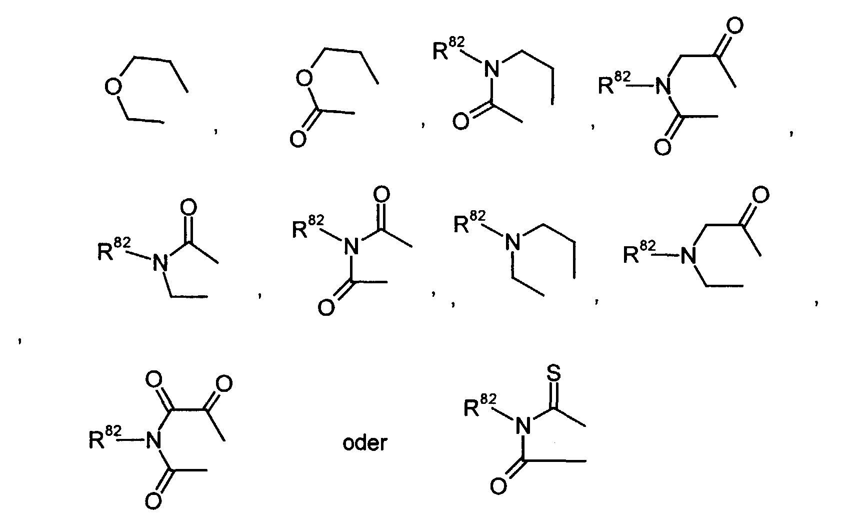 stehen,
in welcher
R⁸² Wasserstoff, geradkettiges oder verzweigtes Alkyl mit bis zu 3 Kohlenstoffatomen bedeutet, das gegebenenfalls durch Cyano, Methoxycarbonyl, Ethoxycarbonyl, Amino, N,N-Dimethylamino oder durch Phenyl substituiert ist, oder geradkettiges oder verzweigtes Acyl mit bis zu 3 Kohlenstoffatomen bedeutet, das gegebenenfalls durch Hydroxy, Amino oder N,N-Dimethylamino substituiert ist, oder Methoxycarbonyl bedeutet,
E für Wasserstoff oder Fluor steht,
R⁷¹ für Wasserstoff, Fluor steht,
und tautomere Formen und/oder Salze davon.

6. Verbindungen der allgemeinen Formel (I) nach Anspruch 1,
in welchen
A für Reste der Formeln 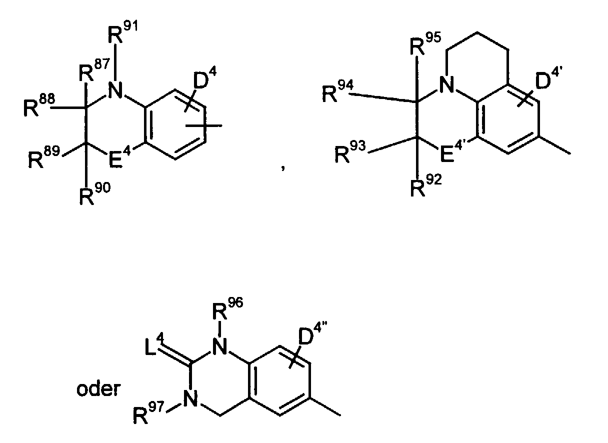 steht,
worin
D⁴, D⁴'und D⁴''gleich oder verschieden sind und Wasserstoff bedeuten,
E⁴ und E⁴' gleich oder verschieden sind und die -CH₂-Gruppe, ein Sauerstoff-oder Schwefelatom oder einen Rest der Formel -SO oder -SO₂ bedeuten,
L⁴ ein Sauerstoff- oder Schwefelatom oder eine Gruppe der Formel - NR⁹⁸ bedeutet,
worin
R⁹⁸ Wasserstoff, Hydroxy oder geradkettiges oder verzweigtes Alkoxy mit bis zu 4 Kohlenstoffatomen bedeutet,
R⁸⁷, R⁸⁸, R⁸⁹, R⁹⁰, R⁹², R⁹³, R⁹⁴ und R⁹⁵ gleich oder verschieden sind und Wasserstoff oder geradkettiges oder verzweigtes Alkyl mit bis zu 3 Kohlenstoffatomen bedeuten, das gegebenenfalls durch Phenyl oder Naphthyl substituiert ist, die ihrerseits ein- bis mehrfach durch Methoxy, Fluor oder Chlor substituiert sein können,
R⁸⁷ und R⁸⁸, R⁸⁹ und R⁹⁰, R⁹² und R⁹³ und/oder R⁹⁴ und R⁹⁵ gemeinsam Gruppen der Formel =O, =CH₂ oder =CHR⁹⁹ bilden,
worin
R⁹⁹ Phenyl oder Pyridyl bedeutet, wobei die Ringsysteme gegebenenfalls ein- bis mehrfach durch Fluor, Chlor, oder durch Methoxy substituiert sind,
R⁹¹, R⁹⁶ und R⁹⁷ gleich oder verschieden sind und Wasserstoff, geradkettiges oder verzweigtes Alkyl oder Alkoxycarbonyl mit jeweils bis zu 3 Kohlenstoffatomen bedeuten, oder
einen Rest der Formel -CO-R¹⁰⁰ bedeuten,
worin
R¹⁰⁰ Phenyl, Naphthyl, Pyridyl, Thienyl, Furyl, Imidazolyl, Pyridazolyl, Pyrimidyl oder geradkettiges oder verzweigtes Alkyl mit bis zu 3 Kohlenstoffatomen bedeutet, das gegebenenfalls durch Phenyl oder Naphthyl substituiert ist, wobei die unter R¹⁰⁰ aufgeführten Ringsysteme gegebenfalls ein- bis mehrfach gleich oder verschieden durch Fluor, Chlor, Brom, Trifluormethyl, Nitro, Hydroxy oder durch geradkettiges oder verzweigtes Alkyl oder Alkoxy mit jeweils bis zu 3 Kohlenstoffatomen substituiert sind,
sowie tautomere Formen und/oder Salze davon.

7. Verbindungen der allgemeinen Formel (I) nach Anspruch 6,
in welchen
A für einen Rest der Formel 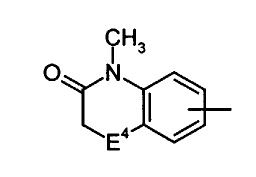 steht, worin
E⁴ ein Sauerstoff- oder Schwefelatom bedeutet, oder die CH₂-Gruppe bedeutet,
und Tautomere und/oder Salze davon.

8. Verbindungen der allgemeinen Formel (I) nach Anspruch 1, worin A eine Gruppe der Formel 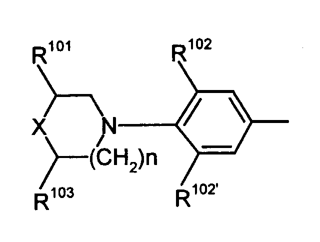 ist,
worin
X O ist; R¹⁰¹ und R¹⁰³ Wasserstoff ist, und,
R¹⁰² und R¹⁰² 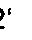 unabhängig voneinander Wasserstoff, Chlor oder Fluor bedeuten,
R¹⁰³ Wasserstoff ist,
n 1 ist,
und Tautomere und/oder Salze davon.

9. Verbindungen der allgemeinen Formel (I) nach irgendeinem der vorgehenden Ansprüche 1 bis 8, dadurch gekennzeichnet, daß R¹ ausgewählt wird aus Vinyl, Ethinyl, Methyl, Ethyl, n-Propyl, Allyl, Benzyl, Phenyl, Trifluormethyl, und Difluormethyl.

10. Verfahren zur Herstellung der Verbindungen der allgemeinen Formel (I) nach Anspruch 1, 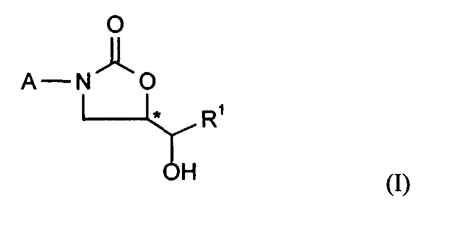 das umfaßt:
[A] die Umsetzung der Verbindungen der allgemeinen Formel (II)
A-NH₂ (II)
mit enantiomerenreinen oder racemischen Verbindungen der allgemeinen Formel (III) 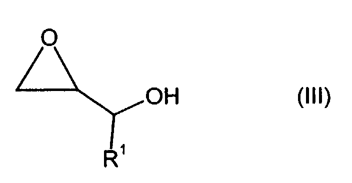 worin A und R¹ die im Anspruch 1 angegebene Bedeutung besitzen, in inerten Lösemitteln und in Anwesenheit eines Hilfsmittels zu enantiomerenreinen oder racemischen Verbindungen der Formel (IV) 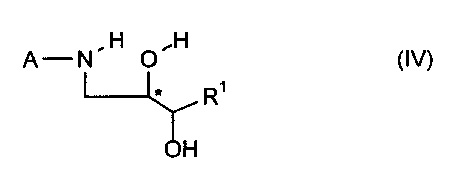 die weiter mit Carbonyldiimidazol in inerten Lösemitteln zu enantiomerenreinen oder racemischen Verbindungen der allgemeinen Formel (I) cyclisiert werden,
oder
[B] die Umsetzung von Verbindungen der Formel (V) 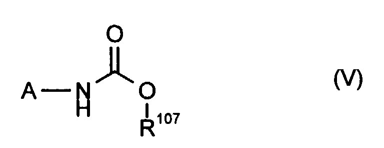 worin A die im Anspruch 1 angegebene Bedeutung aufweist und R¹⁰⁷ wahlweise substituiertes, vorzugsweise durch 1 bis 3 Halogenatome substituiertes (C₁-C₆)Alkyl, wie z.B. Methyl, Ethyl, Propyl oder Trifluormethyl, Phenyl, substituiertes Phenyl, bevorzugt 2- oder 4-Nitrophenyl, oder bevorzugt Benzyl ist, mit enantiomerenreinen oder racemischen Verbindungen der allgemeinen Formel (VI) 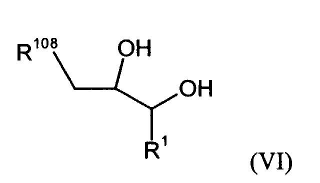 worin R¹ die im Anspruch 1 angegebene Bedeutung aufweist und R¹⁰⁸ eine übliche Abgangsgruppe ist, wie z.B. Halogen, wie Chlor oder Brom, oder -O-SO₂-R¹⁰⁹, worin R¹⁰⁹ (C₁ -C₆)Alkyl, oder gegebenfalls (C₁-C₆)alkylsubstituiertes Phenyl ist,
in Gegenwart von Basen zu Verbindungen der allgemeinen Formel (I) und gegebenfalls anschließend die Umwandlung am Rest R¹ in z.B. Ester oder Amide nach üblichen Methoden.

11. Pharmazeutische Zusammensetzung, die eine Verbindung gemäß irgend einem der Ansprüche 1 bis 9 in Mischung mit einem pharmazeutisch verträglichen Träger bzw. Exzipienten umfaßt.

12. Verbindung nach irgend einem der Ansprüche 1 bis 9 zur Verwendung als Arzneimittel.

13. Verwendung einer Verbindung nach irgend einem der Ansprüche 1 bis 9 zur Herstellung eines Arzneimittels zur Behandlung bakterieller Infektionen.
